# EUROPEAN PATENT SPECIFICATION

(11) **EP 1 539 929 B1**
(45) Date of publication and mention of the grant of the patent: **10.04.2013**
(21) Application number: 03761997.0
(22) Date of filing: 24.06.2003
(51) Int. Cl.: C12N 5/00, C12N 5/02, A01N 63/00, A61K 39/395, A61K 39/40, A61K 39/42, A61K 35/26, A61K 35/28

(54) **METHODS FOR RESTORING IMMUNE REPERTOIRE IN PATIENTS WITH IMMUNOLOGICAL DEFECTS RELATED TO AUTOIMMUNITY AND ORGAN OR HEMATOPOIETIC STEM CELL TRANSPLANTATION**
VERFAHREN ZUR WIEDERHERSTELLUNG DES IMMUNREPERTOIRS IN PATIENTEN MIT IMMUNOLOGISCHEN DEFEKTEN IN VERBINDUNG MIT AUTOIMMUNITÄT SOWIE TRANSPLANTATION VON ORGANEN ODER HÄMATOPOETISCHEN STAMMZELLEN
TECHNIQUES PERMETTANT DE RESTAURER LE REPERTOIRE IMMUNOLOGIQUE DE PATIENTS QUI PRESENTE DES DEFAUTS IMMUNOLOGIQUES LIES A L'AUTO-IMMUNITE ET A UN ORGANE OU A UNE TRANSPLANTATION DE CELLULES SOUCHE HEMATOPOIETIQUES MULTIPOTENTES

(30) Priority: 28.06.2002 US 393042 P; 04.12.2002 US 431212 P; 22.01.2003 US 442001 P
(43) Date of publication of application: 15.06.2005
(73) Proprietor: Life Technologies Corporation, Carlsbad, CA 92008 (US)
(72) Inventor: BERENSON, Ronald, Mercer Island, WA 98040 (US); BONYHADI, Mark, Issaquah, WA 98029 (US); KALAMASZ, Dale, Redmond, WA 98052 (US)
(74) Representative: Weber, Birgit
(86) International application number: PCT/US2003/019842
(87) International publication number: WO 2004/003142

(56) References cited:
- EP-A1- 1 241 249
- WO-A1-02/087627
- WO-A2-03/020904
- US-A1- 2003 119 185
- SHIBUYA T Y ET AL: "ANTI-CD3/ANTI-CD28 BEAD STIMULATION OVERCOMES CD3 UNRESPONSIVENESS IN PATIENTS WITH HEAD AND NECK SQUAMOUS CELL CARCINOMA" ARCHIVES OF OTOLARYNGOLOGY HEAD AND NECK SURGERY, AMERICAN MEDICAL ASSOCIATION, US, vol. 126, no. 4, April 2000 (2000-04), pages 473-479, XP009045082 ISSN: 0886-4470
- LUM L G ET AL: "Immune modulation in cancer patients after adoptive transfer on anti-CD3/anti-CD28-costimulated T cells-phase I clinical trial" JOURNAL OF IMMUNOTHERAPY, LIPPINCOTT WILLIAMS & WILKINS, HAGERSTOWN, MD, US, vol. 24, no. 5, 2001, pages 408-419, XP002957619 ISSN: 1524-9557
- MULLER ET AL: "Reduction of CD3-mediated apoptosis in human T cells by CD28-costimulation: Possible mechanisms" EUROPEAN JOURNAL OF CANCER, PERGAMON PRESS, OXFORD, GB, vol. 33, June 1997 (1997-06), page S35, XP005064560 ISSN: 0959-8049
- MULLER ET AL: "Induction of apoptosis and anergy in resting human T-lymphocytes after CD3-triggering and its modulation by CD28 and cytokines" EUROPEAN JOURNAL OF CANCER, PERGAMON PRESS, OXFORD, GB, vol. 31, no. 1003, October 1995 (1995-10), page S34, XP005250244 ISSN: 0959-8049
- BONYHADI M. ET AL: 'Expansion of Antigen-Specific CTL Using CD3/CD28 Paramagnetic Microbeads (Xcellate TM Beads). For Adoptive Cellular Therapy of Melanoma' BLOOD vol. 98, no. 11, 16 November 2001, pages 32B - 33B, XP002982063
- HEIMFELD S. ET AL: 'Improvements in Gene Therapy' BRITISH JOURNAL OF HAEMATOLOGY vol. 87, no. 1, 1994, page 193, XP008048073

## Description

### BACKGROUND OF THE INVENTION

### Field of the Invention

The present invention relates generally to methods to eliminate undesired (e.g. autoreactive, alloreactive, pathogenic) subpopulations of T cells from a mixed population ofT cells.

### Description of the Related Art

The ability of T cells to recognize the universe of antigens associated with various cancers or infectious organisms is conferred by its T cell antigen receptor (TCR), which is made up of both an α (alpha) chain and a β (beta) chain or a γ (gamma) and a δ (delta) chain. The proteins which make up these chains are encoded by DNA, which employs a unique mechanism for generating the tremendous diversity of the TCR. This multisubunit immune recognition receptor associates with the CD3 complex and binds to peptides presented by the major histocompatibility complex (MHC) class I and II proteins on the surface of antigen-presenting cells (APCs). Binding of TCR to the antigenic peptide on the APC is the central event in T cell activation, which occurs at an immunological synapse at the point of contact between the T cell and the APC.

To sustain T cell activation, T lymphocytes typically require a second co-stimulatory signal. Co-stimulation is typically necessary for a T helper cell to produce sufficient cytokine levels that induce clonal expansion. Bretscher, Immunol. Today 13: 74, 1992; June et al., Immunol. Today 15:321, 1994. The major co-stimulatory signal occurs when a member of the B7 family ligands (CD80 (B7.1) or CD86 (B7.2)) on an activated antigen-presenting cell (APC) binds to CD28 on a T cell.

Methods of stimulating the expansion of certain subsets of T cells have the potential to generate a variety of T cell compositions useful in immunotherapy. Successful immunotherapy can be aided by increasing the reactivity and quantity of T cells by efficient stimulation. Furthermore, in the settings of autoimmunity or transplantation, successful immunotherapy can be aided by the elimination of unwanted autoreactive or alloreactive cells.

The various techniques available for expanding human T cells have relied primarily on the use of accessory cells and/or exogenous growth factors, such as interleukin-2 (IL-2). IL-2 has been used together with an anti-CD3 antibody to stimulate T cell proliferation, predominantly expanding the CD8⁺ subpopulation of T cells. Both the APC signals directed towards the TCR/CD3 complex and CD28 on the surface of T cells are thought to be required for optimal T cell activation, expansion, and long-term survival of the T cells upon re-infusion. The requirement for MHC-matched APCs as accessory cells presents a significant problem for long-term culture systems because APCs are relatively short-lived. Therefore, in a long-term culture system, APCs must be continually obtained from a source and replenished. The necessity for a renewable supply of accessory cells is problematic for treatment of immunodeficiencies in which accessory cells are affected. In addition, when treating viral infection, if accessory cells carry the virus, the cells may contaminate the entire T cell population during long-term culture.

In the absence of exogenous growth factors or accessory cells, a co-stimulatory signal may be delivered to a T cell population, for example, by exposing the cells to a CD3 ligand and a CD28 ligand attached to a solid phase surface, such as a bead. *See* C. June, et al. (U.S. Patent No. 5,858,358); C. June et al. WO 99/953823. While these methods are capable of achieving therapeutically useful T cell populations, increased robustness and ease ofT cell preparation remain less than ideal.

Methods previously available in the art have made use of anti-CD3 and anti CD28 for the expansion of T cells. Shibuya et al (Archives of Otolaryngology head and neck surgery, American Medical Association, US, Vol. 126, No 4, 2000) discloses an *ex-vivo* method of simulating T cells to overcome tumour-related immunosupression. Lum et al (Journal of Immunotherapy, Lippincott, Williams & Wilkins, Hagerstown, MD, US, Vol 24, No. 5, 2001, P408-419) discloses a similar process to that of Shibuya *et al,* where the aim is to activate anti-tumour T cells by exposing them to immobilized anti-CD3 and anti-CD28 antibodies. In addition, the methods currently available in the art have not focused on short-term expansion of T cells or obtaining a more robust population of T cells and the beneficial results thereof. None of these methods has described using such or similar methods to eliminate an undesired clonal or oligoclonal T cell population from a T cell population nor the beneficial results thereof. Moreover, the methods previously available tend to further skew the clonality of the T cell population rather than eliminate undesired reactive clones from a T cell population, and restore a normal immune repertoire. For maximum *in vivo* effectiveness, theoretically, an *ex vivo*, or in vivo-generated, activated T cell population should be in a state that can maximally orchestrate an immune response to cancer, infectious disease, or other disease states. In the setting of autoimmunity or transplantation, the activated T cell populations should be in a state to reconstitute a normal T cell repertoire with a reduced presence or entirely without the presence of autoreactive or potentially pathogenic alloreactive T cells. Currently, patients with autoimmune diseases are treated with long-term immunosuppression to inhibit the autoreactive T cells that cause disease. When the immunosuppressive agents are stopped, disease recurs often concomitant with reappearance of disease causing T cells that re-emerge in these patients. The major problem in hematopoietic stem cell transplantation is graft-versus-host disease (GVHD), which is caused by alloreactive T cells present in the infused hematopoietic stem cell preparation. In organ transplantation, graft rejection mediated by alloreactive host T cells is the major problem, usually overcome by long-term immunosuppression of the transplant recipient.

The present invention provides methods to generate an increased number of more highly activated and more pure T cells that have surface receptor and cytokine production characteristics that appear more healthy and natural than other expansion methods and further provides for the diminution or elimination of undesired autoreactive or alloreactive populations of T cells. The present invention is useful in providing populations of T cells for use in the setting of autoimmune diseases, hematopoietic stem cell, and organ transplantation, as well as other settings where reconstitution of an ablated, abrogated, or otherwise dysfunctional T cell immune system is desired.

Additionally, it is becoming well recognized that the aging immune system is characterized by a progressive decline in the responsiveness to exogenous antigens and tumors in combination with a paradoxical increase in autoimmunity (C. Weyand et al. Mechanisms of Ageing and Development 102;131-147, 1998; D. Schmidt et al. Molecular Medicine 2:608-618, 1996; G. Liuzzo et al. Circulation 100:2135-2139, 1999). These studies have described that aging is associated with the emergence of a subset of T helper cells that are characterized by the loss of CD28 expression. CD4⁺ CD28- T cells are long lived, typically undergo clonal expansion *in vivo,* and react to auto-antigens *in vitro.* The loss of CD28 expression is correlated with a lack of CD40 ligand expression rendering these CD4⁺ T cells incapable of promoting B cell differentiation and immunoglobulin secretion. Aging-related accumulation of CD4⁺ CD28⁻ T cells results in an immune compartment that is skewed towards autoreactive responses and away from the generation of high-affinity B cell responses against exogenous antigens.

### BRIEF SUMMARY OF THE INVENTION

One aspect of the present invention provides for an *ex vivo* method for eliminating at least a substantial portion of a clonal T cell population from a mixed population of T cells from an individual, comprising, exposing a population of cells wherein at least a portion thereof comprises T cells to one or more pro-apoptotic or growth inhibiting compositions wherein said exposure induces apoptosis or growth inhibition in at least a substantial portion of at least one clonal T cell population present in the mixed population of T cells; thereby eliminating at least a substantial portion of said clonal T cell from the mixed population, characterized in that the pro-apoptopic or growth inhibiting composition comprises an autoantigen.

In one embodiment, the method further comprises expanding the mixed population of T cells, by exposing the remaining mixed population of T cells to the pro-apoptotic composition, wherein said exposure induces proliferation in the mixed population of T cells. In one particular embodiment, the pro-apoptotic composition comprises anti-CD3 and anti-CD28 antibodies co-immobilized on a bead. In certain embodiments, the pro-apoptotic composition used to eliminate at least a substantial portion of said clonal T cell population from the mixed population of T cells is the same composition used to expand the remaining mixed population ofT cells.

In one embodiment, the method further comprises expanding the remaining population of cells. In another embodiment, the method further comprises expanding the remaining population of cells by exposing the remaining population of cells to a surface wherein the surface has attached thereto one or more agents that ligate a cell surface moiety of at least a portion of the remaining T cells and stimulates said remaining T cells. In a related embodiment, the surface has attached thereto a first agent that ligates a first T cell surface moiety of a T cell, and the same or a second surface has attached thereto a second agent that ligates a second moiety of said T cell, wherein said ligation by the first and second agent induces proliferation of said T cell.

In one embodiment, the agent attached to the surface is an antibody or an antibody fragment. In another embodiment, the first agent is an antibody or a fragment thereof, and the second agent is an antibody or a fragment thereof In one embodiment the first and the second agents are different antibodies. In one particular embodiment, the first agent is an anti-CD3 antibody, an anti-CD2 antibody, or an antibody fragment of an anti-CD3 or anti-CD2 antibody. In another embodiment, the second agent is an anti-CD28 antibody or antibody fragment thereof. In a further embodiment, the first agent is an anti-CD3 antibody and the second agent is an anti-CD28 antibody.

In another embodiment, the cells are exposed to the surfaces of the present invention for a time sufficient to increase polyclonality. In certain embodiments, this increase in polyclonality comprises a shift from mono to oligoclonality or to polyclonality of the T cell population as measured by a Vβ, Vα, Vγ or Vδ spectratype profile of at least one Vβ, Vα, Vγ or Vδ family gene.

Illustrative pro-apoptotic compositions of the present invention include but are not limited to anti-CD3 antibody, anti-CD2 antibody, anti-CD28 antibody, anti-CD20 antibody, target antigen, MHC-peptide tetramers, Fas ligand, anti-Fas antibody, IL-2, IL-4, TRAIL, rolipram, doxorubicin, chlorambucil, fludarabine, cyclophosphamide, azathioprine, methotrexate, cyclosporine, mycophenolate, FK506, inhibitors of bcl-2, topoisomerase inhibitor, interleukin-1β converting enzyme (ICE)-binding agents, Shigella IpaB protein, staurosporine, ultraviolet irradiation, gamma irradiation, tumor necrosis factor, target antigens nucleic acid molecules, proteins or peptides, and non-protein or non-polynucleotide compounds. In certain embodiments, one or more of these compositions are used at the same time.

In the present invention, the pro-apoptotic compositions comprises an autoantigen. Illustrative autoantigens of the present invention include but are not limited to, myelin basic protein (MBP), MBP 84-102, MBP 143-168, pancreatic islet cell antigens, collagen, CLIP-170, thyroid antigens, nucleic acid, acetylcholine receptor, S Antigen, and type II collagen.

The present invention may be useful in methods for treating autoimmune disease in a patient comprising administering to a patient the populations of T cells generated by the present invention, for example where the patient has been treated with a chemotherapeutic agent prior to administering the population of T cells. Illustrative chemotherapeutic agents include but are not limited to campath, anti-CD3 antibodies, cytoxin, fludarabine, cyclosporine, FK506, mycophenolic acid, steroids, FR901228, and irradiation. In another example, the patient is treated with a T cell ablative therapy prior to administration of the populations of T cells generated by the present invention.

One aspect of the present invention is an *ex vivo* method for eliminating at least a substantial portion of a clonal T cell population from a population of T cells, comprising, providing a population of cells wherein at least a portion thereof comprises T cells; exposing the population of cells to one or more agents that sensitize at least a portion of the T cells to further activation or stimulation, exposing the population of cells to a surface wherein the surface has attached thereto one or more agents that ligate a cell surface moiety of at least a portion of the sensitized T cells and stimulates said sensitized T cells, wherein the exposure of said sensitized T cells to said surface is for a time sufficient to induce apoptosis of said sensitized T cells; thereby eliminating said sensitized T cells from the population, characterized in that the composition that sensitizes comprises an autoantigen. In one embodiment, the method further comprises exposing said population of cells to said surface for a time sufficient to stimulate at least a portion of the remaining T cells and wherein said at least a portion of the remaining cells proliferates. In a further embodiment, the method provides that said surface has attached thereto a first agent that ligates a first T cell surface moiety of a T cell; and the same or a second surface has attached thereto a second agent that ligates a second moiety of said T cell, wherein said ligation by the first and second agent induces proliferation of said T cell. In one embodiment, at least one agent is an antibody or an antibody fragment. In another embodiment, the first agent is an antibody or a fragment thereof, and the second agent is an antibody or a fragment thereof. In yet another embodiment, the first and the second agents are different antibodies. In a related embodiment, the first agent is an anti-CD3 antibody, an anti-CD2 antibody, or an antibody fragment of an anti-CD3 or anti-CD2 antibody. In yet another embodiment, the second agent is an anti-CD28 antibody or antibody fragment thereof. In another embodiment, the first agent is an anti-CD3 antibody and the second agent is an anti-CD28 antibody.

In a related embodiment, cells are exposed to said surface for a time sufficient to increase polyclonality In another embodiment, the increase in polyclonality comprises a shift from mono to oligoclonality or to polyclonality of the T cell population as measured by a Vβ, Vα, Vγ or Vδ spectratype profile of at least one Vβ, Vα, Vγ or Vδ family gene.

In certain embodiments, the patient requires a hematopoietic stem cell transplant. In a related embodiment, the composition that sensitizes recipient PBMCs that have been treated such that they are unable to continue dividing and the population of cells comprises donor T cells. The present invention may be useful in methods for reducing the risk of, or the severity of, an adverse GVHD effect in a patient who is undergoing a hematopoietic stem cell transplant, comprising administering to said patient the population of T cells produced according to the methods described herein. In certain embodiments, the patients to receive the cells require an organ transplant. In a related embodiment the composition that sensitizes comprises irradiated donor cells and the population of cells comprises recipient T cells. These cells may be administered to a patient receiving an organ transplant, to reduce the risk of organ rejection. For example, the organ transplant patient is treated with a T cell ablative therapy prior to administration of the population of T cells.

In one aspect of the present invention the composition that sensitizes comprises an autoantigen. Illustrative autoantigens of the present invention include but are not limited to myelin basic protein (MBP), MBP 84-102, MBP 143-168, pancreatic islet cell antigens, S Antigen, and type II collagen. A patient with an autoimmune disease may be treated by administration of a population of T cells generated according to this method. The patient may be treated with a T cell ablative therapy prior to administering the population of T cells.

The purity of the CD3+/CD28⁺ T cells may be at least 90% pure. In further embodiments, the purity of the CD3⁺/CD28⁺ T cells is 91%, 92%, 93%, 94%, 95%, 96%, 97%, or 98% pure. The purity of the CD3⁺/CD28⁺T cells may be at least 99% pure. The purity of the CD3⁺/CD28⁺ T cells may be at least 99.9% pure. The population of CD3⁺/CD28⁺ T cells may comprise less than 9%, 8%, 7%, 6%, 5%, 4%, 3%, 2%, 1%, 0.5% or 0.1% contaminating CD28⁻T cells.

In one embodiment the CD3⁺ surface molecule is stimulated using anti-CD3 antibodies and the CD28⁺ surface molecule is stimulated using anti-CD28 antibodies.

A substantially pure population of CD3⁺CD28⁺ T cells, including CD4⁺CD3⁺CD28⁺ T cells, and CD8⁺CD3⁺CD28⁺ T cells can be used in the treatment of people suffering from autoimmune diseases such as, rheumatoid arthritis, multiple sclerosis, insulin dependent diabetes, Addison's disease, celiac disease, chronic fatigue syndrome, inflammatory bowel disease, ulcerativecolitis, Crohn's disease, Fibromyalgia, systemic lupus erythematosus, psoriasis, Sjogren's syndrome, hyperthyroidism/Graves disease, hypothyroidism/Hashimoto's disease, Insulin-dependent diabetes (type 1), Myasthenia Gravis, endometriosis, scleroderma, pernicious anemia, Goodpasture syndrome, Wegener's disease, glomerulonephritis, aplastic anemia, paroxysmal nocturnal hemoglobinuria, myelodysplastic syndrome, idiopathic thrombocytopenic purpura, autoimmune hemolytic anemia, Evan's syndrome, Factor VIII inhibitor syndrome, systemic vasculitis, dermatomyositis, polymyositis and rheumatic fever.

### BRIEF DESCRIPTION OF THE SEVERAL VIEWS OF THE DRAWINGS

Figure 1 is a dot plot showing the presence of CD3+CD8+ HLA-A2CMVpp65 antigen specific T cells in an HLA-A2-positive donor.
Figure 2 is a dot plot showing an increase in CD25 expression in CMV-activated HLA-A2CMVpp65 antigen-specific T cells.

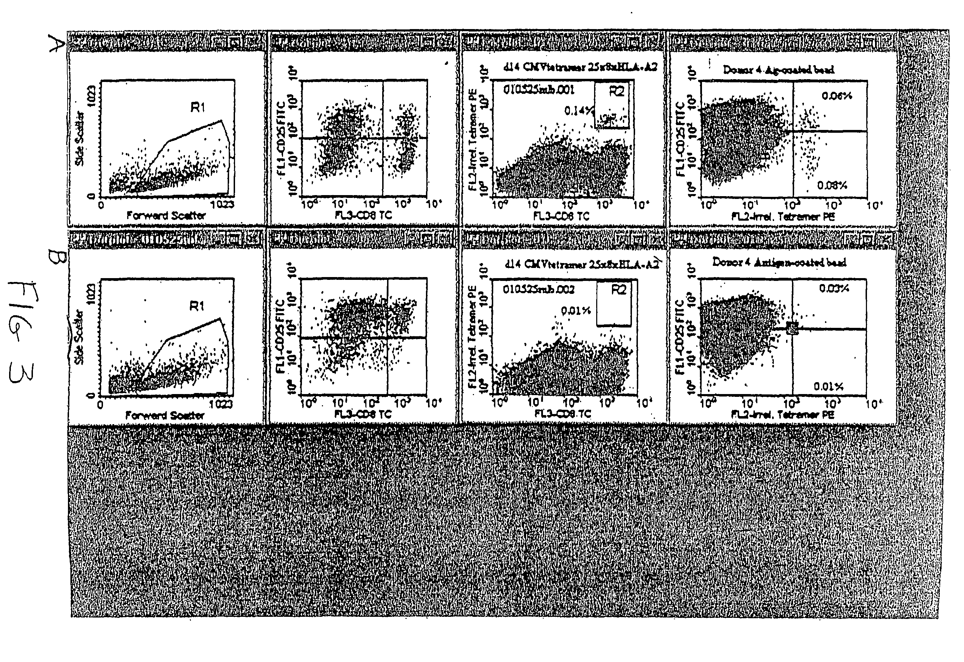

Figure 3 is a dot plot showing the upregulation of CD25 on restimulated cells, and the deletion of prestimulated tetramer-positive cells (i.e., CMVpp65-Agspecific) by the secondary strong stimulation provided by the 3X28 beads. At day 14 post-primary stimulation, cultures were either left unstimulated (Panels A1-A4) or were restimulated using the XCELLERATE™ process with 3X28 beads for 16 hours (Panels B1-B4). CD25 is upregulated on restimulated cells (Panel B2), but tetramer-positive (*i.e*., CMVpp65-Ag-specific) prestimulated cells were deleted by the secondary strong stimulation provided by the 3X28 beads (Panel B3).
Figure 4 is a histogram showing the increase in expression of key effector molecules, including CD95, on leukemic B-cells co-cultured with XCELLERATED T cells™.
Figure 5 is a dot plot showing the induction of apoptosis in leukemic B-cells co-cultured with XCELLERATED T cells™.
Figure 6 is a graph showing the disappearance of leukemic B-cells during the XCELLERATE™ process and the concomitant expansion of T cells.
Figure 7 is a graph comparing fold increase of polyclonal T cells to the fold increase of CMV pp65 A2-tetramer+ (antigen-specific) T cells using varying bead:cell ratios. Solid bars represent polyclonal T cells. Striped bars represent CMV-specific T cells.

### DETAILED DESCRIPTION OF THE INVENTION

Prior to setting forth the invention, it may be helpful to an understanding thereof to set forth definitions of certain terms that will be used hereinafter.

The term "biocompatible", as used herein, refers to the property of being predominantly non-toxic to living cells.

The term "stimulation", as used herein, refers to a primary response induced by ligation of a cell surface moiety. For example, in the context of receptors, such stimulation entails the ligation of a receptor and a subsequent signal transduction event. With respect to stimulation of a T cell, such stimulation refers to the ligation of a T cell surface moiety that in one embodiment subsequently induces a signal transduction event, such as binding the TCR/CD3 complex. Further, the stimulation event may activate a cell and up- or down-regulate expression of cell surface molecules such as receptors or adhesion molecules, or up- or down-regulate secretion of a molecule, such as downregulation of Tumor Growth Factor beta (TGF-β). Thus, ligation of cell surface moieties, even in the absence of a direct signal transduction event, may result in the reorganization of cytoskeletal structures, or in the coalescing of cell surface moieties, each of which could serve to enhance, modify, or alter subsequent cell responses.

The term "activation", as used herein, refers to the state of a cell following sufficient cell surface moiety ligation to induce a measurable morphological, phenotypic, and/or functional change. Within the context of T cells, such activation may be the state of a T cell that has been sufficiently stimulated to induce cellular proliferation. Activation of a T cell may also induce cytokine production and/or secretion, and up- or down-regulation of expression of cell surface molecules such as receptors or adhesion molecules, or up- or down-regulation of secretion of certain molecules, and performance of regulatory or cytolytic effector functions. Within the context of other cells, this term infers either up- or down-regulation of a particular physico-chemical process.

The term "target cell", as used herein, refers to any cell that is intended to be stimulated by cell surface moiety ligation.

An "antibody", as used herein, includes both polyclonal and monoclonal antibodies (mAb); primatized (e.g., humanized); murine; mouse-human; mouse-primate; and chimeric; and may be an intact molecule, a fragment thereof (such as scFv, Fv, Fd, Fab, Fab' and F(ab)'₂ fragments), or multimers or aggregates of intact molecules and/or fragments; and may occur in nature or be produced, e.g., by immunization, synthesis or genetic engineering; an "antibody fragment," as used herein, refers to fragments, derived from or related to an antibody, which bind antigen and which in some embodiments may be derivatized to exhibit structural features that facilitate clearance and uptake, *e.g.,* by the incorporation of galactose residues. This includes, *e.g.,* F(ab), F(ab)'₂, scFv, light chain variable region (V_{L}), heavy chain variable region (V_{H}), and combinations thereof.

The term "protein", as used herein, includes proteins, glycoproteins and other cell-derived modified proteins, polypeptides and peptides; and may be an intact molecule, a fragment thereof, or multimers or aggregates of intact molecules and/or fragments; and may occur in nature or be produced, *e.g*., by synthesis (including chemical and/or enzymatic) or genetic engineering.

The term "agent", "ligand", or "agent that binds a cell surface moiety", as used herein, refers to a molecule that binds to a defined population of cells. The agent may bind any cell surface moiety, such as a receptor, an antigenic determinant, or other binding site present on the target cell population. The agent may be a protein, peptide, antibody and antibody fragments thereof, fusion proteins, synthetic molecule, an organic molecule (*e.g*., a small molecule), or the like. Within the specification and in the context of T cell stimulation, antibodies are used as a prototypical example of such an agent.

The term "cell surface moiety" as used herein may refer to a cell surface receptor, an antigenic determinant, or any other binding site present on a target cell population.

The terms "agent that binds a cell surface moiety" and "cell surface moiety", as used herein, should be viewed as a complementary/anti-complementary set of molecules that demonstrate specific binding, generally of relatively high affinity.

A "co-stimulatory signal", as used herein, refers to a signal, which in combination with a primary signal, such as TCR/CD3 ligation, leads to T cell proliferation and/or activation.

"Separation", as used herein, includes any means of substantially purifying one component from another (*e.g*., by filtration, affinity, buoyant density, or magnetic attraction).

A "surface", as used herein, refers to any surface capable of having an agent attached thereto and includes, without limitation, metals, glass, plastics, copolymers, colloids, lipids, cell surfaces, and the like. Essentially any surface that is capable of retaining an agent bound or attached thereto.

"Monoclonality", as used herein, in the context of a population of T cells, refers to a population of T cells that has a single specificity as defined by spectratype analysis (a measure of the TCR Vβ, Vα, Vγ, or Vδ chain hypervariable region repertoire). A population of T cells is considered monoclonal (or mono-specific) when the Vβ, Vα, Vγ, and/or Vδ spectratype profile for a given TCR Vβ, Vα, Vγ, and/or Vδ family has a single predominant peak. Spectratype analysis distinguishes rearranged variable genes of a particular size, not sequence. Thus, it is understood that a single peak could represent a population of T cells expressing any one of a limited number of rearranged TCR variable genes (Vβ, Vα, Vγ, or Vδ) comprising any one of the 4 potential nucleotides (adenine (a), guanine (g), cytosine (c), or thymine (t)) or a combination of the 4 nucleotides at the junctional region. In certain embodiments of the present invention, it may be desirable to clone and sequence a particular band to determine the sequence(s) of the rearranged variable gene(s) present in the band representing a particular length..

"Oligoclonality", as used herein, in the context of a population of T cells, refers to a population of T cells that has multiple, but narrow antigen specificity. This can be defined by spectratype analysis (a measure of the TCR Vβ, Vα, Vγ, or Vδ) chain hypervariable region repertoire). A population of T cells is considered oligoclonal when the Vβ spectratype profile for a given TCR Vβ, Vα, Vγ, or Vδ family has between about 2 and about 4 predominant peaks. This can also be defined by generation and characterization of antigen-specific clones to an antigen of interest.

"Polyclonality", as used herein, in the context of a population of T cells, refers to a population of T cells that has multiple and broad antigen specificity. This can be by spectratype analysis (a measure of the TCR Vβ, Vα, Vγ, or Vδ chain hypervariable region repertoire). A population of T cells is considered polyclonal when the Vβ spectratype profile for a given TCR Vβ, Vα, Vγ, or Vδ family has multiple peaks, typically 5 or more predominant peaks and in most cases with Gaussian distribution. Polyclonality can also be defined by generation and characterization of antigen-specific clones to an antigen of interest.

"Restoring or increasing the polyclonality", as used herein refers to a shift from a monoclonal profile to an oligoclonal profile or to a polyclonal profile, or from an oligoclonal profile to a polyclonal profile, in expressed TCR Vβ, Vα, Vγ, and/or Vδ genes in a population of T cells, as measured by spectratype analysis or by similar analysis such as flow cytometry or sequence analysis. The shift from a monoclonal Vβ, Vα, Vγ, and/or Vδ expression profile in a population of T cells to an oligoclonal profile or to a polyclonal profile is generally seen in at least one TCR Vβ, Vα, Vγ, and/or Vδ family. In one embodiment of the present invention, this shift is observed in 2, 3, 4, or 5 Vβ families. In certain embodiments of the present invention, a shift is observed in 6, 7, 8, 9, or 10 Vβ families. In a further embodiment of the present invention, a shift is observed in from 11, 12, 13, or 14 Vβ families. In a further embodiment of the present invention, a shift is observed in from 15 to 20 Vβ families. In a further embodiment of the present invention, a shift is observed in 20 to 24 Vβ families. In another embodiment, a shift is seen in all Vβ families. The functional significance of restoring or increasing the polyclonality of a population of T cells is that the immune potential, or the ability to respond to a full breadth of antigens, of the population of T cells is restored or increased. In certain aspects of the present invention, some T cells within a population may not have their TCRs engaged by the methods set forth herein (*e.g*., T cells with downregulated TCR expression). However, by being in close proximity to T cells activated by the methods described herein, and the factors secreted by them, these T cells may in turn upregulate their TCR expression thereby resulting in a further increase in the polyclonality of the population of T cells. Restoration or increase in polyclonality can also be measured by determining the breadth of response to a particular antigen of interest, for example by measuring the number of different epitopes recognized by antigen-specific cells. This can be carried out using standard techniques for generating and cloning antigen-specific T cells *in vitro.*

The term "clonal T cell population" as used herein, refers to a T cell population that has a given range of specificities against a given target antigen. This can be measured by any number of assays known in the art, for example by generating and measuring the breadth of specificities (*i.e*. number of different specificities) of antigen-specific clones in a given population. A clonal T cell population can also be defined by having either monoclonal or oligoclonal specificity as defined by spectratype analysis (a measure of the TCR Vβ, Vα, Vγ, or Vδ chain hypervariable region repertoire).

The term "animal" or "mammal" as used herein, encompasses all mammals, including humans. Preferably, the animal of the present invention is a human subject.

The term "exposing" as used herein, refers to bringing into the state or condition of immediate proximity or direct contact.

The term "proliferation" as used herein, means to grow or multiply by producing new cells.

"Immune response or responsiveness" as used herein, refers to activation of cells of the immune system, including but not limited to, T cells, such that a particular effector function(s) of a particular cell is induced. Effector functions may include, but are not limited to, proliferation, secretion of cytokines, secretion of antibodies, expression of regulatory and/or adhesion molecules, and the ability to induce cytolysis.

"Stimulating an immune response" as used herein, refers to any stimulation such that activation and induction of effector functions of cells of the immune system are achieved.

"Immune response dysfunction" as used herein, refers to the inappropriate activation and/or proliferation, or lack thereof, of cells of the immune system, and/or the inappropriate secretion, or lack thereof, of cytokines, and/or the inappropriate or inadequate induction of other effector functions of cells of the immune system, such as expression of regulatory, adhesion, and/or homing receptors, and the induction of cytolysis.

"Particles" or "surface" as used herein, may include a colloidal particle, a microsphere, nanoparticle, a bead, or the like. A surface may be any surface capable of having a ligand bound thereto or integrated into, including cell surfaces (for example K562 cells), and that is biocompatible, that is, substantially non-toxic to the target cells to be stimulated. In the various embodiments, commercially available surfaces, such as beads or other particles, are useful (*e.g*., Miltenyi Particles, Miltenyi Biotec, Germany; Sepharose beads, Pharmacia Fine Chemicals, Sweden; DYNABEADS™, Dynal Inc., New York; PURABEADS™, Prometic Biosciences, magnetic beads from Immunicon, Huntingdon Valley, PA, microspheres from Bangs Laboratories, Inc., Fishers, IN).

"Paramagnetic particles" as used herein, refer to particles, as defined above, that localize in response to a magnetic field.

A "pro-apoptotic composition" "apoptotic compositions" or "inducer of apoptosis", as used herein refers to any composition or stimulus that increases the apoptotic activity of a cell either when administered alone or in conjunction with other pro-apoptotic compositions. The pro-apoptotic compositions used in the methods of the present invention preferably induce apoptosis in activated T cells, NKT, NK or B-cells. In certain embodiments, a pro-apoptotic composition of the present invention will induce apoptosis without further activation/stimulation. Illustrative examples of such compositions or stimuli include, but are not limited to, deprivation of a growth factor, oxidizing conditions, heat stress, serum starvation, phorbol myristate acetate (PMA) and ionomycin, superantigens (*e.g*. SEA, SEB, and the like) various antibodies, such as anti-CD2, anti-CD3, anti-CD28, anti-CD20, anti-Fas antibody, or any combination thereof, MHC-peptide tetramers or dimers, Fas ligand, IL-2, IL-4, TRAIL, rolipram, doxorubicin, chlorambucil, fludarabine, corticosteroids, glucocorticoids, cyclosporine, cyclophosphamide, FK506, azathioprine, methotrexate, mycophenolate, annexin, caspases, inhibitors of bcl-2, topoisomerase inhibitors, interleukin-1β converting enzyme (ICE)-binding agents, Shigella IpaB protein, staurosporine, ultraviolet irradiation, gamma irradiation, radiation, tumor necrosis factor, various histone deacetylase inhibitors, and others well known in the art. In certain embodiments, the pro-apoptotic compositions comprises a surface, such as a magnetic bead, having attached thereto one or more agents that binds a cell surface moiety. In this regard, the agent can be any agent as described herein. In one embodiment, the surface has attached thereto at least anti-CD3 antibodies. In another embodiment, the surface has attached thereto anti-CD3 and anti-CD28 antibodies. In addition, a stimulator of apoptosis can be a polypeptide that is capable of increasing or inducing the apoptotic activity of a cell. Such polypeptides include those that directly regulate the apoptotic pathway such as Bax, Bad, Bcl-xS, Bak, Bik, and active caspases as well as those that indirectly regulate the pathway. In certain embodiments, the pro-apoptotic composition comprises activated T cells, such as XCELLERATED T cells™ (such as those described in U.S. Patent Application No. 10/133,236), in particular for inducing apoptosis in populations of B-cells. Other illustrative pro-apoptotic compositions include, but are not limited to, irradiated cells (*e.g*. donor or recipient (allogeneic) cells), target antigens (*e.g*. defined autoimmune target antigens for example, in multiple sclerosis, the target antigen identified as myelin basic protein (MBP) MBP 84-102, or MBP 143-168; pancreatic islet cell antigens; in uveitis, the S Antigen; or in rheumatoid arthritis, type II or other types of collagen; in Grave's disease, thyroid receptor; in Myasthena gravis, acetylcholine receptor), cytoplasmic linker protein-170 (CLIP-170), nucleic acid molecules, proteins or peptides, and non-protein or non-polynucleotide compounds.

A "composition that sensitizes cells to further activation or stimulation" or "sensitizing composition" as used herein is any composition which sensitizes cells to subsequent activation/stimulation. Upon subsequent activation/stimulation, sensitized cells undergo apoptosis. Sensitizing compositions of the present invention also sensitize cells to the effects of pro-apoptotic compositions. Illustrative compositions that sensitize cells to further activation, stimulation, or the effects of pro-apoptotic compositions include cells that have been treated such that they are unable to continue dividing, for example by irradiation, (*e.g*. donor or recipient (allogeneic) cells), superantigens (*e.g*. SEA, SEB, and the like), target antigens (*e.g*. defined autoimmune target antigens for example, in multiple sclerosis, the target antigen identified as myelin basic protein (MBP) MBP 84-102, or MBP 143-168; pancreatic islet cell antigens; in uveitis, the S Antigen; or in rheumatoid arthritis, type II or other types of collagen; in Grave's disease, thyroid receptor; in Myasthena gravis, acetylcholine receptor, nucleic acid molecules, proteins or peptides, and non-protein or non-polynucleotide compounds), protein, glycoprotein, peptides, antibody/antigen complexes, cell lysate, non-soluble cell debris, apoptotic bodies, necrotic cells, whole cells from a cell line that have been treated such that they are unable to continue dividing, natural or synthetic complex carbohydrates, lipoproteins, transformed cells or cell line, transfected cells or cell line, or transduced cells or cell line, or any combination thereof.

Apoptosis, for purposes of the present invention, is defined as programmed cell death. Apoptosis is a programmed cell death which is a widespread phenomenon that plays a crucial role in the myriad of physiological and pathological processes. Apoptosis occurs in embryogenesis, metamorphosis, endocrine-dependent tissue atrophy, normal tissue turnover, and death of immune thymocytes (induced through their antigen-receptor complex or by glucocorticoids) (Itoh et al., Cell 66:233, 1991). During maturation of T cells in the thymus, T cells that recognize self-antigens are destroyed through the apoptotic process, whereas others are positively selected. The possibility that some T cells recognizing certain self epitopes (*e.g*., inefficiently processed and presented antigenic determinants of a given self protein) escape this elimination process and subsequently play a role in autoimmune diseases has been suggested (Gammon et al., Immunology Today 12:193, 1991). Necrosis is an accidental cell death which is the cell's response to a variety of harmful conditions and toxic substances. Apoptosis, morphologically distinct from necrosis, is a spontaneous form of cell death that occurs in many different tissues under various conditions. Apoptosis occurs in two stages. The cell undergoes nuclear and cytoplasmic condensation, and may eventually break into a number of membrane-bound fragments containing structurally intact apoptotic bodies, which are phagocytosed by neighboring cells and rapidly degraded. Alternatively, cells entering the apoptotic pathway may be phagocytosed prior to degeneration into membrane bound bodies. Apoptosis is observed in many different tissues, healthy and neoplastic, adult and embryonic. Death occurs spontaneously, or is induced by physiological or noxious agents. Apoptosis is a basic physiological process that plays a major role in the regulation of cell populations.

Methods for measuring apoptosis are well known in the art. Apoptosis can be determined by methods such as, for example, DNA ladder, electron or light microscopy, flow cytometry, and different commercially available kits for the determination of apoptosis.

As used herein, a "growth inhibiting composition" is any substance that inhibits growth in cells, or otherwise renders cells dysfunctional and unable to divide either when administered alone or in conjunction with other compositions of the present invention. The growth-inhibiting compositions used in the methods of the present invention preferably inhibit growth in activated T cells, NKT, NK or B-cells. Illustrative examples of such compositions or stimuli include, but are not limited to, but are not limited to, deprivation of a growth factor, oxidizing conditions, heat stress, serum starvation, phorbol myristate acetate (PMA) and ionomycin, superantigens (e.g. SEA, SEB, and the like) various antibodies, such as anti-CD2, anti-CD3, anti-CD28, anti-CD20, anti-Fas antibody, or any combination thereof, MHC-peptide tetramers or dimers, Fas ligand, IL-2, IL-4, TRAIL, rolipram, doxorubicin, chlorambucil, fludarabine, corticosteroids, glucocorticoids, cyclosporine, cyclophosphamide, FK506, azathioprine, methotrexate, mycophenolate, annexin, caspases, inhibitors of bcl-2, topoisomerase inhibitors, interleultin-1β converting enzyme (ICE)-binding agents, Shigella IpaB protein, staurosporine, ultraviolet irradiation, gamma irradiation, radiation, tumor necrosis factor, various histone deacetylase inhibitors, and others well known in the art. In certain embodiments, the growth inhibiting compositions comprises a surface, such as a magnetic bead, having attached thereto one or more agents that binds a cell surface moiety. In this regard, the agent can be any agent as described herein. In one embodiment, the surface has attached thereto at least anti-CD3 antibodies. In another embodiment, the surface has attached thereto anti-CD3 and anti-CD28 antibodies. In addition, a growth inhibiting composition can comprise a polypeptide that is capable of inhibiting growth of a cell. Such polypeptides include those peptides such as Bax, Bad, Bcl-xS, Bak, Bik, and active caspases. Other illustrative growth inhibiting compositions include, but are not limited to, irradiated cells (*e.g.* donor or recipient (allogeneic) cells), target antigens (*e.g*. defined autoimmune target antigens for example, in multiple sclerosis, the target antigen identified as myelin basic protein (MBP) MBP 84-102, or MBP 143-168; pancreatic islet cell antigens; in uveitis, the S Antigen; or in rheumatoid arthritis, type II or other types of collagen; in Grave's disease, thyroid receptor; in Myasthena gravis, acetylcholine receptor), cytoplasmic linker protein-170 (CLIP-170), nucleic acid molecules, proteins or peptides, and non-protein or non-polynucleotide compounds.

As used herein, a "substantially pure" population of CD3⁺/CD28⁺ T cells is a population of cells that is comprised of at least about 90% CD3⁺/CD28⁺ T cells. In certain aspects of the invention a "substantially pure" population of CD3+/CD28+ T cells is a population of cells that is comprised of at least about 91%, 92%, 93%, 94%, 95%, 96%, 97%, or 98% CD3⁺/CD28⁺ T cells, preferably at least about 99%, and even more preferably about 99.9% or more.

### SOURCES OF MIXED POPULATION OF CELLS

In one embodiment, cells to be exposed to the pro-apoptotic or growth inhibiting compositions and/or sensitizing compositions are from the circulating blood of an individual and are obtained from one or more units of blood or from an apheresis or leukapheresis. The apheresis product typically contains lymphocytes, including T cells, monocytes, granulocytes, B cells, other nucleated white blood cells, red blood cells, and platelets. Prior to exposure to a sensitizing composition and subsequent activation and/or stimulation, a source of T cells is obtained from a subject. The term "subject" is intended to include living organisms in which an immune response can be elicited (*e.g*., mammals). Examples of subjects include humans, dogs, cats, mice, rats, and transgenic species thereof. T cells can be obtained from a number of sources, including peripheral blood mononuclear cells, bone marrow, thymus, tissue biopsy, tumor, lymph node tissue, gut associated lymphoid tissue, mucosa associated lymphoid tissue, spleen tissue, or any other lymphoid tissue, and tumors. T cells can be obtained from T cell lines and from autologous or allogeneic sources. T cells may also be obtained from a xenogeneic source, for example, from mouse, rat, non-human primate, and pig. In certain embodiments of the present invention, T cells can be obtained from a unit of blood collected from a subject using any number of techniques known to the skilled artisan, such as ficoll separation. In one preferred embodiment, cells from the circulating blood of an individual are obtained by apheresis or leukapheresis. The apheresis product typically contains lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing steps. In one embodiment of the invention, the cells are washed with phosphate buffered saline (PBS). In an alternative embodiment, the wash solution lacks calcium and may lack magnesium or may lack many if not all divalent cations. As those of ordinary skill in the art would readily appreciate a washing step may be accomplished by methods known to those in the art, such as by using a semi-automated "flow-through" centrifuge (for example, the Cobe 2991 cell processor, Baxter) according to the manufacturer's instructions. After washing, the cells may be resuspended in a variety of biocompatible buffers, such as, for example, calcium (Ca)-free, magnesium (Mg)-free PBS. Alternatively, the undesirable components of the apheresis sample may be removed and the cells directly resuspended in culture media.

In another embodiment, T cells are isolated from peripheral blood lymphocytes by lysing or removing the red blood cells and depleting the monocytes, for example, by centrifugation through a PERCOLL™ gradient. A specific subpopulation of T cells, such as CD28⁺, CD4⁺, CD8⁺, CD45RA⁺, and CD45RO⁺T cells, can be further isolated by positive or negative selection techniques. For example, in one preferred embodiment, T cells are isolated by incubation with anti-CD3/anti-CD28 (*i.e*., 3x28)-conjugated beads, such as DYNABEADS® M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another preferred embodiment, the time period is 10 to 24 hours. In one preferred embodiment, the incubation time period is 24 hours. For isolation of T cells from patients with leukemia, use of longer incubation times, such as 24 hours, can increase cell yield. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. For example, CD3⁺ CD28⁺ T cells can be positively selected using CD3/CD28 conjugated magnetic beads (*e.g*., DYNABEADS® M-450 CD3/CD28 T cell Expander). In one aspect of the present invention, enrichment of a T cell population by negative selection can be accomplished with a combination of antibodies directed to surface markers unique to the negatively selected cells. A preferred method is cell sorting and/or selection via negative magnetic immunoadherence or flow cytometry that uses a cocktail of monoclonal antibodies directed to cell surface markers present on the cells negatively selected. For example, to enrich for CD4⁺ cells by negative selection, a monoclonal antibody cocktail typically includes antibodies to CD14, CD20, CD1 lb, CD16, HLA-DR, and CD8.

The present invention may enable the provision of a T cell population or composition that has been depleted or enriched for populations of cells expressing a variety of markers, such as CD62L, CD45RA or CD45RO, cytokines (*e.g*. IL-2, IFN-γ, IL-4, IL-10), cytokine receptors (*e.g*. CD25), perforin, adhesion molecules (*e.g*. VLA-1, VLA-2, VLA-4, LPAM-1, LFA-1), and/or homing molecules (*e.g*. L-Selectin), prior to sensitization, stimulation and expansion. Cells expressing any of these markets are depleted or positively selected by antibodies or other ligands/binding agents directed to the marker. One of ordinary skill in the art would readily be able to identify a variety of particular methodologies for depleting or positively selecting for a sample of cells expressing a desired marker.

Monocyte populations (*i.e*., CD114⁺ cells) may be depleted from blood preparations prior to *ex vivo* expansion by a variety of methodologies, including anti-CD 14 coated beads or columns, or utilization of the phagocytotic activity of these cells to facilitate removal or through adherence to plastic. Accordingly, in one embodiment, the invention uses paramagnetic particles of a size sufficient to be engulfed by phagocytotic monocytes. In certain embodiments, the paramagnetic particles are commercially available beads, for example, those produced by Dynal AS under the trade name DYNABEADS™. Exemplary DYNABEADS™ in this regard are M-280, M-450, and M-500. In one aspect, other non-specific cells are removed by coating the paramagnetic particles with "irrelevant" proteins (*e.g*., serum proteins or antibodies). Irrelevant proteins and antibodies include those proteins and antibodies or fragments thereof that do not specifically target the T cells to be expanded. In certain embodiments the irrelevant beads include beads coated with sheep anti-mouse antibodies, goat anti-mouse antibodies, and human serum albumin.

In brief such depletion of monocytes is performed by preincubating PBMC that have been isolated from whole blood using Ficoll, or apheresed peripheral blood with one or more varieties of irrelevant or non-antibody coupled paramagnetic particles at any amount that allows for removal of monocytes (approximately a 20:1 bead:cell ratio)for about 30 minutes to 2 hours at 22 to 37 degrees C, followed by magnetic removal of cells which have attached to or engulfed the paramagnetic particles. Preincubation can also be done at temperatures as low as 3-4 degrees C. Such separation can be performed using standard methods available in the art. For example, any magnetic separation methodology may be used including a variety of which are commercially available, (*e.g*., DYNAL^{®} Magnetic Particle Concentrator (DYNAL MPC^{®})). Assurance of requisite depletion can be monitored by a variety of methodologies known to those of ordinary skill in the art, including flow cytometric analysis of CD14 positive cells, before and after said depletion.

T cells for exposure to pro-apoptotic and/or sensitizing compositions and subsequent stimulation may also be frozen after the washing step, which does not require the monocyte-removal step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing a final concentration of 10% DMSO and 4% human serum albumin, or other suitable cell freezing media, the cells then are frozen to -80°C at a rate of 1° per minute and stored in the vapor phase of a liquid nitrogen storage tank.

### ELIMINATION OF UNDESIRED SUBPOPULATIONS OF CELLS FROM A

### MIXED POPULATION OF CELLS

### Direct Exposure to Pro-Apoptotic Compositions

The present invention provides for methods to eliminate at least a portion of undesired clonal populations of T cells from a population of immune cells.

Undesired populations of cells can be eliminated or reduced by a statistically significant amount directly through the exposure of said cells to a pro-apoptotic composition. Exposure to the pro-apoptotic composition can take place *in vivo* or *in vitro*. Without being bound by theory, the previously activated cells are thought to be more sensitive to apoptotic compositions than naive or unactivated cells. Therefore, exposure to apoptotic compositions either *in vivo* or *in vitro*, using doses and conditions that induce apoptosis, will selectively kill highly activated cells such as unwanted autoreactive cells in a patient.

Thus, the present invention provides methods for the elimination of at least a substantial portion of any unwanted subpopulation of clonal T cells from a mixed population of immune cells. For the purposes of the present invention, a substantial portion means at least 70% of the unwanted subpopulation of cells. In certain embodiments, a substantial portion means 75%, 80%, 85%, 90%, 95%, 96%, 97%, 98%, 99% and higher of the unwanted subpopulation of cells. Elimination of cells can be measured using any number of techniques known in the art, including but not limited to flow cytometric analysis using a variety of antibodies and/or peptide-MHC tetramers and functional assays such as proliferation and chromium release assays.

Pro-apoptotic compositions or inducers of apoptosis refers to any composition or stimulus that increases the apoptotic activity of a cell either when administered alone or in conjunction with other pro-apoptotic compositions. The pro-apoptotic compositions used in the methods of the present invention preferably induce apoptosis in activated T cells, NKT cells, NK cells, and B cells. The amount and conditions under which the pro-apoptotic compositions induce desired apoptosis may vary and can be determined by the skilled artisan using routine optimization. In certain embodiments, a pro-apoptotic composition of the present invention will induce apoptosis without further activation/stimulation. Illustrative examples of such agents or stimuli include, but are not limited to, deprivation of a growth factor, oxidizing conditions, heat stress, freeze-thaw stress, serum starvation, various antibodies, such as anti-CD2, anti-CD3, anti-CD28, anti-CD20, or anti-Fas antibody; MHC-peptide tetramers; Fas ligand, TRAIL, FR901228 (as described in U.S. Patent No. 6,403,555), FK506, annexin, caspases, cytokines such as IL-2 or IL-4, cyclophosphamide, chemotherapeutic agents, UV, steroids, corticosteroids, glucocorticoids, rolipram, doxorubicin, chlorambucil, fludarabine, inhibitors of bcl-2, topoisomerase inhibitors, interleukin-1β converting enzyme (ICE)-binding agents, Shigella IpaB protein, staurosporine, ultraviolet irradiation, gamma irradiation, radiation, tumor necrosis factor, histone deacetylase inhibitors, and others well known in the art. In certain embodiments, the pro-apoptotic composition comprises a surface, such as a magnetic bead, having attached thereto one or more agents that binds a cell surface moiety. In this regard, the agent can be any agent as described herein. In one embodiment, the surface has attached thereto at least anti-CD3 antibodies. In another embodiment, the surface has attached thereto anti-CD3 and anti-CD28 antibodies. In addition, a stimulator of apoptosis can be a polypeptide that is capable of increasing or inducing the apoptotic activity of a cell. Such polypeptides include those that directly regulate the apoptotic pathway such as Bax, Bad, Bcl-xL, Bak, Bik, and active caspases as well as those that indirectly regulate the pathway. Other illustrative pro-apoptotic compositions include, but are not limited to, irradiated cells (*e.g*. donor or recipient (allo) cells), target antigens (*e.g*. defined autoimmune target antigens such as myelin basic protein (MBP), pancreatic islet cell antigens, cytoplasmic linker protein-170 (CLIP-170), Sjogren's syndrome antigen A (SS-A/Ro), Sjogren's syndrome antigen B (SS-B/La), Sjogren's lupus antigen (SL), scleroderma antigen 70 (Scl-70)) nucleic acid molecules, proteins or peptides, and non-protein or non-polynucleotide compounds.

As the skilled artisan will readily recognize, tests on any pro-apoptotic composition used in the methods of the present invention would need to be routinely carried out over a range of doses to determine: 1) the behavior of these substances; and 2) safety and identification of any untoward effects 3) optimal doses for effective induction of apoptosis in cells to be eliminated. Thus, the particular pro-apoptotic compositions employed in the methods described herein would require individual routine optimization. In one particular embodiment, the population of remaining cells that has been cleared of unwanted reactive subpopulations of cells can then be administered to the patient without further stimulation/activation or expansion.

In one embodiment of the present invention, cells are exposed to pro-apoptotic compositions multiple times either alone or in combination with other pro-apoptotic compositions. In certain aspects of the present invention, it may be preferable to activate/stimulate and in some cases also expand a mixed population of cells as described below in the sections entitled "Stimulation/Activation of Cell Populations" and "Expansion of Cell Populations" prior to exposure to one or more pro-apoptotic compositions. In one preferred embodiment, the cells remaining in the population following exposure to a pro-apoptotic compositions of the present invention, are activated/stimulated and expanded *in vitro* as described below in the sections entitled "Stimulation/Activation of Cell Populations" and "Expansion of Cell Populations". In certain embodiments, the pro-apoptotic composition and the composition used to activate/stimulate and expand are the same composition. In one particular embodiment, a surface having attached thereto an agent, as described herein, is used as a pro-apoptotic composition and further, used to active/stimulate and expand a mixed population. In this regard, certain clonal cells in the population are induced to undergo apoptosis while others are stumulated/activated and proliferate in response to the composition. In this context, an illustrative composition that can be used both to induce apoptosis in a subpopulation of T cells and to stimulate/activate and expand a mixed population of T cells comprises anti-CD3 and anti-CD28 antibodies co-immobilized on beads (3x28 beads).

In another embodiment of the present invention, the cells remaining following exposure to one or more pro-apoptotic compositions, are further stimulated/activated and expanded *in vivo*. *In vivo* stimulation and expansion of the cells of the present invention can be carried out using any number of cytokines, such as IL-2 and IL-4 or other agents described herein that simulate cells.

In a further embodiment of the present invention, the cells remaining following exposure to one or more pro-apoptotic compositions, are further stimulated/activated and expanded *in vitro* using the surfaces and agents bound thereto as described below in the sections entitled "Stimulation/Activation of Cell Populations" and "Expansion of T cell Populations". The stimulation and activation of the remaining cells that have not undergone apoptosis using the surfaces of the present invention, can increase polyclonality of said remaining population of T cells as measured by the breadth of the response of the population to a given antigen. Restoration or increase in polyclonality can be measured by determining the breadth of response to a particular antigen of interest, for example by measuring the number of different epitopes recognized by antigen-specific cells. This can be carried out using standard techniques for generating and cloning antigen-specific T cells *in vitro.*

The stimulation and activation using the surfaces of the present invention of the remaining cells that have not undergone apoptosis, restores polyclonality to said remaining population of T cells with respect to expressed TCR genes as indicated by spectratype analysis. Polyclonality of the T cell compositions of the present invention are as described in U.S. Patent Application No. 60/375,733. Spectratype analysis is a method for measuring TCR Vβ, Vα, Vγ, or Vδ gene usage by a pool of T cells and levels of nucleotide insertion during the recombination process in T cell development (as described in U.S. Patent No. 5,837,447). Spectratype analysis can be used to measure the breadth or narrowness of the T cell immune response potential. Additionally, spectratype analysis can be used to determine if specific undesired clonal populations of T cells have been removed from a mixed population of T cells.

The ability of V, D, and J gene segments to combine together randomly introduces a large element of combinatorial diversity into the TCR repertoire. The precise point at which V, D, and J segments join can vary, giving rise to local amino acid diversity at the junction. The exact nucleotide position of joining can differ by as much as 10 residues resulting in deletion of nucleotides from the ends of the V, D, and J gene segments, thereby producing codon changes at the junctions of these segments. Diversity is further increased during the rearrangement process when additional nucleotides not encoded by either gene segment are added at the junction between the joined gene segments. (The variability created by this process is called "N-region diversity.") (Janeway, Travers, Walport. Immunobiology. Fourth Ed., 98 and 150. Elsevier Science Ltd/Garland Publishing. 1999).

The level of diversity for the T cell repertoire can be measured, in part, by evaluating which TCR Vβ, Vα, Vγ, or Vδ chains are being employed by individual T cells within a pool of circulating T cells, and by the number of random nucleotides inserted next to the Vβ gene at the V-D-J or V-J gene junctions. In general, when the circulating T cell pool contains T cells expressing the full range of TCR Vβ, Vα, Vγ, or Vδ chains and when those individual V region chains are derived from gene recombination events which utilize the broadest array of inserted nucleotides, the T cell arm of the immune system will have its greatest potential for recognizing the universe of potential antigens. When the range of TCR V region chains expressed by the circulating pool of T cells is limited or reduced, and when expressed TCRs utilize chains encoded by recombined genes with limited nucleotide insertions, the breadth of the immune response potential is correspondingly reduced. The consequences of this are a reduced ability to respond to the wide variety of antigens leading to increased risks of infection and cancer.

Methods for determining apoptosis are known in the art and are described, for example, in Current Protocols in Immunology, John Wiley & Sons, New York, N.Y., or in U.S. Patent No. 6,312,684. Illustrative assays to measure apoptosis comprise DNA ladder, electron or light microscopy, flow cytometry, and different commercially available kits for the determination of apoptosis. In certain embodiments, cells are observed for morphological changes, such as chromatin condensation, cell shrinkage, increased granularity and other indicia of apoptosis known to those of skill in the art. Chromatin condensation can be detected by standard methods, such as light microscopy of stained cell preparations. Cell shrinkage and granularity can be readily detected by measuring the light scattering properties of the cells (Kerr, et al. supra., and Wyllie, et al., supra). Observation of single or double stranded fragmentation of DNA into oligonucleosomal ladders often is another indication that apoptosis has been induced (Arend, et al., Am. J. Pathol, 136:593, 1990; Wyllie, et al., J. Pathol, 142.:67, 1984). Sometimes, however, apoptotic cells do not exhibit double stranded internucleosomal DNA fragmentation (Collins, et al., Int. J. Rad. Biol., 62:45 1992; Cohen, et al., Biochem. J., 286:331 1992); instead, single DNA strand breaks will be observed. Single-strand breaks can readily be detected using a method of *in situ* nick end-labeling of the DNA. This method is described by Wyllie, et al. (Br. J. Cancer, 67:20,1993).

In one embodiment, the cells are exposed to a growth inhibiting composition as described herein. In certain embodiments, the growth inhibiting compositions of the present invention inhibit growth in at least a substantial portion of at least one clonal population of T cells such that when a mixed population of cells is activated/stimulated and expanded as described herein, the growth inhibited cells do not expand and are eventually out-competed by the mixed population of cells. The end result of this being the effective elimination of the growth inhibited cells from the mixed population of cells.

### Exposure to Compositions That Sensitize Cells to Further Stimulation/Activation

Alternatively, at least a substantial portion of an undesired population of cells can be eliminated by first sensitizing the cells to further stimulation/activation and then further simulating or activating them by exposure to a surface of the present invention. This additional stimulation/activation induces apoptosis in the sensitized cells, leading to their elimination from the population. The sensitizing compositions also sensitize cells to the effects of pro-apoptotic compositions described above. Thus, the present invention provides for methods to eliminate at least a substantial portion of an undesired clonal population of T cells from a population of immune cells by exposure to one or more compositions that sensitize the undesired populations of cells to further stimulation/action or to the effects of a pro-compositions comprising populations of cells that no longer contain undesired cells, and uses thereof.

In one aspect of the present application, a population of T cells is exposed to a composition or compositions that sensitize to further activation or stimulation, at least a portion of cells, e.g. previously highly activated T cells. In a preferred embodiment, the sensitized cells comprise undesired autoreactive T cells. In a further embodiment, the sensitized cells comprise alloreactive cells present in donor hematopoietic stem cell. In yet a further embodiment, the sensitized cells comprise alloreactive cells from a potential organ transplant recipient.

In one embodiment, the sensitizing composition comprises irradiated cells. In a particular embodiment, the irradiated cells are from a hematopoietic stem cell transplant recipient and the cells to be sensitized are from the hematopoietic stem cell transplant donor. In another embodiment, the sensitizing composition comprises irradiated cells from an organ donor and the cells to be sensitized are cells from the organ recipient. In certain embodiments, the cells to be sensitized are cells from an organ recipient post-transplant. Cells are typically irradiated with gamma rays in the range of about 3000 to 3600 rads, more preferably at about 3300 rads. Other irradiated cells that may be useful in the present invention, such as lymphoblastoid or tumor cell lines are typically irradiated with gamma rays in the range of about 6000 to 10,000 rads, more preferably at about 8000 rads. Cells may also be treated by other means such as with chemical agents (e.g., etiposide, mitomycin, and the like).

Sensitizing compositions include any composition or combination of compositions that sensitizes T cells, to subsequent stimulation such that subsequent stimulation or activation induces apoptosis. Sensitizing compositions of the present invention also include any composition or combination of compositions that sensitizes T cells, to subsequent exposure to a pro-apoptotic composition. Sensitizing compositions include but are not limited to antibodies such as anti-CD2, anti-CD3, anti-FAS; MHC-peptide dimers or tetramers, cytokines such as IL-2, TRAIL, compounds such as rolipram, doxorubicin, chlorambucil and fludarabine. Sensitizing compositions also include FAS-ligand and the natural ligands for CD2 and CD3. Sensitizing compositions also include inhibitors of bc1-2, such as those described in U.S. Patent No. 6,277,844, topoisomerase inhibitors, such as etoposide, CPT-11 and topotecan, and others, such as described in U.S. Patent No. 5,834,012. Other illustrative sensitizing compositions include interleukin-113 converting enzyme (ICE)-binding agents that induce apoptosis, such as Shigella IpaB protein described in U.S. Patent No. 5,972,899, or compounds described in U.S. Patent Nos. 6,350,741, 6,294,546 and 6,329,365.

Sensitizing compositions comprise autoantigens. Autoantigens may be defined autoimmune target antigens e.g. defined autoimmune target antigens for example, in multiple sclerosis, the target antigen identified as myelin basic protein (MBP) MBP 84-102, or MBP 143-168; pancreatic islet cell antigens; in uveitis, the S Antigen; or in rheumatoid arthritis, type II or other types of collagen; in SLE, cytoplasmic linker protein-170 (CLIP-170); Sjogren's syndrome antigen A (SS-A/Ro), Sjogren's syndrome antigen B (SS-B/La), Sjogren's lupus antigen (SL); scleroderma antigen 70 (Scl-70); in Grave's disease, thyroid receptor; in Myasthena gravis, acetylcholine receptor, nucleic acid molecules, proteins or peptides, and non-protein or non-polynucleotide compounds. Autoantigens also comprise peptide mixtures eluted from MHC molecules known to be associated with autoimmunity, for example, HLA-DQ and -DR molecules that confer susceptibility to several common autoimmune diseases, such as type 1 diabetes, rheumatoid arthritis and multiple sclerosis, or HLA-B27 molecules known to confer susceptibility to reactive arthritis and ankylosing spondylitis. Autoantigens may also be synthesized peptides predicted to bind to MHC molecules associated with autoimmune diseases.

Sensitizing compositions may be used for selectively eliminating at least a substantial portion of a population of T cells expressing a specific Vβ, Vα, Vγ or Vδ gene. For example, antibodies specific for a particular Vβ, Vα, Vγ or Vδ gene can be used to specifically sensitize the T cells according to the methods of the present invention. Alternatively, T cells expressing a particular Vβ, Vα, Vγ or Vδ gene of interest can be negatively selected, thereby eliminating at least a substantial portion of them from a population.

In further embodiments, one or more sensitizing compositions are used simultaneously and for times sufficient to induce the desired sensitization.

Ample evidence from the development of vaccines suggests that either synthetic peptides or recombinant DNA-derived proteins are effective in eliciting an immune response in humans. These studies also provide guidance as to the range of doses effective for immunization (Zajoc, B.A., D.J. West, W.J. McAleer and E.M. Scolnick, Overview of clinical studies with Hepatitis B vaccine made by recombinant DNA, J. Infect. 13:(Suppl A)39-45 (1986). Yamamoto, S., T. Kuroki, K. Kurai and S. lino, Comparison of results for phase I studies with recombinant and plasma-derived hepatitis B vaccines, and controlled study comparing intramuscular and subcutaneous injections of recombinant hepatitis B vaccine, J. Infect. 13:(Suppl A)53-60 (1986). Francis, D.P. et al., The prevention of Hepatitis B with vaccine, Ann. Int. Med. 97:362-366 (1982). Putney et al., Features of HIV envelope and development of a subunit vaccine, AIDS Vaccine Research and Clinical Trials, S. Putney and B. Bolognesi, eds. (New York: Dekker) pp. 3-62 (1990). Steven, V.C. and W.R. Jones, Vaccines to prevent pregnancy, New Generation Vaccines, G.C. Woodrow and M.M. Levine, eds. (New York: Dekker) pp. 879-900 (1990). Herrington et al., Safety and immunogenicity in man of a synthetic peptide malaria vaccine against Plasmodium Falciparium sporozoites, Nature, 328:257-259 (1987)).

In one embodiment, immunization (*i.e. in vivo* sensitization) with an agent that sensitizes cells to further stimulation/activation or exposure to a pro-apoptotic composition, is then followed by a waiting period during which the agent activates the subset of cells bearing reactive receptors, such as T cells bearing reactive TCRs, causing them to express cytokine receptors, such as the IL-2 receptor. For example, this process will induce IL-2 receptors only on T cells that have been antigenically-stimulated. Based on studies of both human and mouse T cells *in vitro*, between about 12 to about 24 hour after antigen exposure are required to express significant numbers of IL-2 receptors, and as long as about 72 hours are required to express optimal numbers of IL-2 receptors on the majority of T cells. Thus, the waiting period can be as short as about 12 hours or as long as about 72 hours, and in the case of various disease states, due to retarded immune responsiveness, this period may be as long as 120 hours, becoming increasingly optimal toward the upper end of this range.

In one embodiment, IL-2, or other appropriate cytokines, such as IL-4, are administered to induce apoptosis in the activated cells as described above. Administration of IL-2 to humans has been well-studied in cancer patients, and various doses have been evaluated (Loize, M.T., L.W. Frana, S.O. Sharrow, R.J. Robb and S.A. Rosenberg, In vivo administration of purified human interleukin 2. I. Half-life and immunologic effects of the Jurkat cell line-derived interleukin 2. J. Immunol. 134:157-166 (1985). Lotze, J.T., Y.L. Malory, S.E. Ettinghausen, A.A. Rayner, S.O. Sharrow, C.A.Y. Seipp, M.C. Custer and S.A. Rosenberg, In vivo administration of purified human interleukin 2. II. Half-life, immunologic effects, and expansion of peripheral lymphoid cells in vivo with recombinant IL 2. J. Immunol. 135:2865-2875 (1985). Donahue, J.H. and S.A. Rosenberg, The fate of interleukin-2 after in vivo administration, J. Immunol. 130:2203-2208 (1983). Belldegrun, A., M.M. Muul and S.A Rosenberg, Interleukin 2 expanded tumor-infiltrating lymphocytes in human renal cell cancer: isolation, characterization, and antitumor activity, Cancer Research 48:206-214 (1988). Rosenberg, S.A., M.T. Lotze, L.M. Muul, S. Leitman, A.E. Chang, S.E. Ettinghausen, Y.L. Malory, J.M. Skibber, E. Shiloni, J.T. Vetto, C.A. Seipp, C. Simpson and C.M. Reichert, Observations on the systemic administration of autologous lymphokine-activated killer cells and recombinant interleukin-2 to patients with metastatic cancer, New Eng. J. Med. 313:1485-1492 (1985).). Data indicate that IL-2 should be given I.V., either as frequent bolus doses or as a continuous infusion. Doses that have been previously established range between about 300 to about 3000 units/kg/hour continuous infusion, or from 104 to 106 units/kg I.V. bolus.

The population of cells may be exposed to one or more sensitizing compositions *in vitro.* As the skilled artisan will readily recognize, tests on any sensitizing composition used in the methods would need to be routinely carried out over a range of doses to determine: 1) the pharmacokinetic behavior of these substances; and 2) safety and identification of any untoward effects 3) optimal doses for effective induction of apoptosis in cells to be eliminated. Thus, the particular sensitizing compositions employed in the methods described herein would require individual routine optimization.

### Stimulation of Sensitized Cells to Induce Apoptosis of Cells to be Eliminated From a Mixed Population of Cells

In one aspect, a population of immune cells comprising sensitized cells as described above is further activated or stimulated to induce apoptosis as described below in the section entitled "Stimulation/Activation of Cell Populations", thereby eliminating the sensitized T cells, from the mixed population of cells. At the same time, the desired cells that remain, *e.g*., those cells that are not sensitized to undergo apoptosis, are activated and stimulated to expand, thereby resulting in a population of activated cells from which at least a substantial portion of unwanted subpopulations of T have been eliminated. As mentioned previously, stimulation/activation as described herein may be carried out on cells remaining following exposure of a mixed population of cells directly to pro-apoptotic compositions. Furthermore, the subsequent stimulation and activation provided by the present invention restores polyclonality to the population of T cells with respect to expressed TCR genes as indicated by spectratype analysis.

In one embodiment, sensitized cells are stimulated/activated as described below multiple times with or without additional sensitizing composition, as many times as is necessary to eliminate at least a substantial portion of the undesired cells. For example in the setting of an autoimmune disease, the present invention provides for methods to stimulate cells a second or more times in the presence of antigen (*i.e*., sensitizing composition) after the initial round of stimulation/activation. Likewise, in the setting of hematopoietic stem cell transplantation, the present application provides for methods to stimulate cells from the hematopoietic stem cell donor a second or more times, or as many times as necessary to eliminate at least a substantial portion of the undesired cells, in the presence of irradiated cells from a hematopoietic stem cell transplant recipient. In the setting of organ transplantation, the present application provides for methods to stimulate cells from the organ recipient a second or more times, or as many times as is necessary to eliminate at least a substantial portion of undesired cells, if necessary in the presence of irradiated cells from the organ donor. In one embodiment, the methods are carried out on cells from a patient (*e.g*. host cells) post-transplant in order to eliminate undesired cells. In certain embodiments, it may not be necessary to eliminate all of the undesired cells, for example in the setting of hematopoietic stem cell transplantation for certain types of cancer, graft versus leukemic cell effect may be desired.

In certain aspects, it may be preferable to stimulate/activate and in some cases expand a mixed population of cells as described below in the sections entitled "Stimulation/Activation of Cell Populations" and "Expansion of Cell Populations" prior to exposure to one or more agents that sensitize cells to further stimulation/activation and subsequent stimulation.

In further aspects, the cells are sensitized and then exposed to a pro-apoptotic composition, thereby eliminating at least a substantial portion of cells that have become sensitized to the effects of the pro-apoptotic composition. The cells remaining in the population can then be further stimulated/activated and expanded as described below.

In one embodiment, the cells are exposed to a growth inhibiting composition as described herein. In certain embodiments, the growth inhibiting compositions inhibit growth in at least a substantial portion of at least one clonal population of T cells such that when a mixed population of cells is activated/stimulated and expanded as described herein, the growth inhibited cells do not expand and are eventually out-competed by the mixed population of cells. The end result of this being the effective elimination of the growth inhibited cells from the mixed population of cells.

### Generation of a Pure CD3⁺CD28⁺ T Cell Population

A pure population of CD3⁺CD28⁺ T cells can be generated by magnetic concentration, selection, and stimulating the mixed population of T cells with a composition capable of stimulating both CD3 and CD28 molecules on the surface of a T cell. Selection and stimulation of both the CD3 and CD28 molecules on the surface of a cell results in the activation and proliferation of this subset of cells. Conversely, under conditions described herein, exposure of a CD3⁺ CD28 T cell to a composition capable of selecting and stimulating both CD3 and CD28 surface molecules would be insufficient to induce both activation and expansion of this population of T cells. Further, shortening incubation time with CD3/CD28 beads as described herein, favors selection of CD3⁺CD28⁺ cells at the expense of CD3⁺ CD28-cells (*e.g*., a 15 minute selection at 1 r.p.m. at room temperature, followed by magnetic concentration leaves many or most CD3⁺CD28⁻ cells behind.) Triggering of the TCR by either a specific antigen or by a molecule capable of stimulating the CD3 surface molecule, for example an anti-CD3 antibody, is considered insufficient to induce expansion and lymphokine secretion unless supplemented by co-stimulatory signals, *i.e*., the specific stimulation of the CD28 molecule. In fact, in the absence of co-stimulation, these T cells may acquire a state of non-responsiveness or energy.

A population of substantially pure CD3⁺ CD28⁺ T cells contains less than 10% CD3⁺ CD28- T cells. In certain embodiments, a population of substantially pure CD3⁺ CD28⁺ T cells contains less than 9%, 8%, 7%, 6%, 5%, 4%, 3%, 2%,1%, 0.5%, or 0.1% CD3⁺CD28⁻ T cells

Thus, the methods of the present application, *e.g*., the stimulation and selection of a mixed population ofT cells using a composition capable of triggering CD3 and simulating CD28, would result in the generation of a substantially pure population of CD3⁺ CD28⁺ T cells

This population of substantially pure CD3⁺ CD28⁺ T cells can be used to treat acute or chronic GVHD. A population of substantially pure CD3⁺ CD28⁺ T cells can be used to treat autoimmune diseases, such as rheumatoid arthritis, multiple sclerosis, insulin dependent diabetes, Addison's disease, celiac disease, chronic fatigue syndrome, colitis, Crohn's disease, fibromyalgia, lupus, psoriasis, Sjogren's syndrome, hyperthyroidism/Graves disease, hypothyroidism/Hashimoto's disease, insulin-dependent diabetes (type 1), Myasthenia Gravis, endometriosis, scleroderma, pernicious anemia, Goodpasture syndrome, Wegener's disease and rheumatic fever. In a further embodiment, the cells can be used to treat autoimmunity associated with large granular lymphocyte leukemia (LGL). A mixed population of immune cells could be removed from a donor and these cells stimulated with a composition capable of stimulating CD3 and CD28 molecules. While not wanting to be bound by theory, it is postulated that this stimulation results in the specific activation and expansion of CD3⁺CD28⁺ T cells, and result in the anergy of T cells that lack the expression of the co-stimulatory molecule, CD28. Once the pure population of CD3⁺ CD28⁺ T cells has been generated, these cells can then be infused for the treatment of an autoimmune disease, LGL, or GVHD.

### STIMULATION/ACTIVATION OF CELL POPULATIONS

The stimulated and activated T cells are generated by cell surface moiety ligation that induces activation. In certain embodiments, the stimulated and activated T cells are generated by activating a population of T cells solely via engagement of the TCR, for example using anti-CD antibodies or natural ligands for the TCR. In certain embodiments, the stimulated an activated T cells are generated by activating a population of T cells and stimulating an accessory molecule on the surface of the T cells with a ligand which binds the accessory molecule, as described for example, in US patent application numbers 08/253,694, 08/435,816, 08/592,711, 09/183,055, 09/350,202, and 09/252,150, and patent numbers 6,352,694, 5,858,358 and 5,883,223. In the context of sensitized cells described above, activating said sensitized population of T cells and stimulating an accessory molecule on the surface of said sensitized T cells with a ligand which binds the accessory molecule induces apoptosis and subsequent elimination of the cells.

Generally, T cell activation of cells may be accomplished by cell surface moiety ligation, such as stimulating the T cell receptor (TCR)/CD3 complex or the CD2 surface protein. A number of anti-human CD3 monoclonal antibodies are commercially available, exemplary are, clone BC3 (XR-CD3; Fred Hutchinson Cancer Research Center, Seattle, WA), OKT3, prepared from hybridoma cells obtained from the American Type Culture Collection, and monoclonal antibody G19-4. Similarly, stimulatory forms of anti-CD2 antibodies are known and available. Stimulation through CD2 with anti-CD2 antibodies is typically accomplished using a combination of at least two different anti-CD2 antibodies. Stimulatory combinations of anti-CD2 antibodies that have been described include the following: the T11.3 antibody in combination with the T11.1 or T11.2 antibody (Meuer et al., Cell 36:897-906, 1984), and the 9.6 antibody (which recognizes the same epitope as T11.1) in combination with the 9-1 antibody (Yang et al., J. Immunol. 137:1097-1100, 1986). Other antibodies that bind to the same epitopes as any of the above described antibodies can also be used. Additional antibodies, or combinations of antibodies, can be prepared and identified by standard techniques. Stimulation may also be achieved through contact with superantigens (*e.g*., *Staphylococcus* enterotoxin A (SEA), *Staphylococcus* enterotoxin B (SEB), Toxic Shock Syndrome Toxin 1 (TSST-1)), endotoxin, or through a variety of mitogens, including but not limited to, phytohemagglutinin (PHA), phorbol myristate acetate (PMA) and ionomycin, lipopolysaccharide (LPS), T cell mitogen, and IL-2.

To further activate a population of T cells, a co-stimulatory or accessory molecule on the surface of the T cells, such as CD28, is stimulated with a ligand that binds the accessory molecule. Accordingly, one of ordinary skill in the art will recognize that any agent, including an anti-CD28 antibody or fragment thereof capable of cross-linking the CD28 molecule, or a natural ligand for CD28 can be used to stimulate T cells. Exemplary anti-CD28 antibodies or fragments thereof useful in the context of the present application include monoclonal antibody 9.3 (IgG2ₐ) (Bristol-Myers Squibb, Princeton, NJ), monoclonal antibody KOLT-2 (IgG1), 15E8 (IgG1), 248.23.2 (IgM), clone B-T3 (XR-CD28; Diaclone, Besançon, France) and EX5.3D10 (IgG2ₐ) (ATCC HB11373). Exemplary natural ligands include the B7 family of proteins, such as B7-1 (CD80) and B7-2 (CD86) (Freedman et al., J. Immunol. 137:3260-3267, 1987; Freeman et al., J. Immunol. 143:2714-2722, 1989; Freeman et al., J. Exp. Med. 174:625-631, 1991; Freeman et al., Science 262:909-911, 1993; Azuma et al., Nature 366:76-79, 1993; Freeman et al., J. Exp. Med. 178:2185-2192, 1993).

Other illustrative accessory molecules on the surface of the T cells that can be stimulated with a ligand that binds the accessory molecule include, but are not limited to, CD54, LFA-1, ICOS, and CD40.

In addition, binding homologues of a natural ligand, whether native or synthesized by chemical or recombinant techniques, can also be used in accordance with the present application. Other agents may include natural and synthetic ligands. Agents may include, but are not limited to, other antibodies or fragments thereof, growth factor, cytokine, chemokine, soluble receptor, steroid, hormone, mitogen, such as PHA, or other superantigens.

As described earlier, the subsequent stimulation and activation of the remaining cells that have not undergone apoptosis or have not been sensitized to undergo apoptosis, restores polyclonality to said remaining population of T cells with respect to expressed TCR genes as indicated by spectratype analysis.

### EXPANSION OF CELL POPULATIONS

Generally, the present application provides for expansion of the population of cells that remains following exposure of the population to a pro-apoptotic compositions or a sensitizing composition and any subsequent induction of apoptosis in undesired subpopulations of cells, preferably autoreactive or undesired alloreactive T cells. In one embodiment, the remaining T cells may be stimulated by a single agent. In another embodiment, remaining T cells are stimulated with two or more agents, one that induces a primary signal and additional agents that induce one or more co-stimulatory signals. Ligands useful for stimulating a single signal or stimulating a primary signal and an accessory molecule that stimulates a second signal may be used in soluble form, attached to the surface of a cell, or immobilized on a surface as described herein. A ligand or agent that is attached to a surface serves as a "surrogate" antigen presenting cell (APC). In a preferred embodiment both primary and secondary agents are co-immobilized on a surface. In one embodiment, the molecule providing the primary activation signal, such as a CD3 ligand, and the co-stimulatory molecule, such as a CD28 ligand, are coupled to the same surface, for example, a particle. Further, as noted earlier, one, two, or more stimulatory molecules may be used on the same or differing surfaces.

The cell population may be stimulated as described herein, such as by contact with an anti-CD3 antibody or an anti-CD2 antibody immobilized on a surface, or by contact with a protein kinase C activator (*e.g*., bryostatin) in conjunction with a calcium ionophore. For co-stimulation of an accessory molecule on the surface of the T cells, a ligand that binds the accessory molecule is used. For example, a population of CD4⁺ cells can be contacted with an anti-CD3 antibody and an anti-CD28 antibody, under conditions appropriate for stimulating proliferation of the T cells. Alternatively, a population of cells can be contacted with PMA and ionomycin. Similarly, to stimulate proliferation of CD8⁺ T cells, an anti-CD3 antibody and the anti-CD28 antibody B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):1319-1328, 1999; Garland et al., J. Immunol Meth. 227(1-2):53-63, 1999).

The primary stimulatory signal and the co-stimulatory signal for the T cell may be provided by different protocols. For example, the agents providing each signal may be in solution or coupled to a surface. When coupled to a surface, the agents may be coupled to the same surface (*i.e*., in "cis" formation) or to separate surfaces (*i.e*., in "trans" formation). Alternatively, one agent may be coupled to a
surface and the other agent in solution. In one embodiment, the agent providing the co-stimulatory signal is bound to a cell surface and the agent providing the primary activation signal is in solution or coupled to a surface. In certain embodiments, both agents can be in solution. In another embodiment, the agents may be in soluble form, and then cross-linked to a surface, such as a cell expressing Fc or Sc receptors or an antibody or other binding agent which will bind to the agents. In a preferred embodiment, the two agents are immobilized on a spherical or semi-spherical surface, the prototypic examples being beads or cells, either on the same bead, *i.e*., "cis," or to separate beads, *i.e.,* "trans." By way of example, the agent providing the primary activation signal is an anti-CD3 antibody and the agent providing the co-stimulatory signal is an anti-CD28 antibody; and both agents are co-immobilized to the same bead in equivalent molecular amounts. In one embodiment, a 1:1 ratio of each antibody bound to the beads for T cell expansion and T cell growth is used. In certain aspects of the present invention, a ratio of anti CD3:anti-CD28 antibodies (CD3:CD28) bound to the beads is used such that an increase in T cell expansion is observed as compared to the expansion observed using a ratio of 1:1. In one particular embodiment an increase of from about 0.5 to about 3 fold is observed as compared to the expansion observed using a ratio of 1:1. In one embodiment, the ratio of anti-CD3 :anti-CD28 (CD3 :CD28) antibody bound to the beads ranges from about 100:1 1 to 1:100 and all integer values there between. In certain embodiments, the ratio of CD3:CD28 is at least about 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 9:1, 8:1, 7:1, 6:1, 5:1, 4:1, 3:1, 2:1, or 1:1. In one aspect, more anti-CD28 antibody is bound to the particles than anti-CD3 antibody, *i.e.* the ratio of CD3:CD28 is less than one. In certain embodiments, the ratio of anti-CD28 antibody to anti CD3 antibody bound to the beads is greater than 2:1. In one particular embodiment, a 1:200 CD3:CD28 ratio of antibody bound to beads is used. In one particular embodiment, a 1:150 CD3:CD28 ratio of antibody bound to beads is used. In one particular embodiment, a 1:100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:45 CD3:CD28 ratio of antibody bound to beads is used. In another embodiment, a 1:40 CD3:CD28 ratio of antibody bound to beads is used. In another embodiment, a 1:35 CD3:CD28 ratio of antibody bound to beads is used. In another embodiment, a 1:30 CD3:CD28 ratio of antibody bound to beads is used. In another embodiment, a 1:25 CD3:CD28 ratio of antibody bound to beads is used. In another embodiment, a 1:20 CD3:CD28 ratio of antibody bound to beads is used. In another embodiment, a 1:15 CD3:CD28 ratio of antibody bound to beads is used. In one preferred embodiment, a 1:10 CD3:CD28 ratio of antibody bound to beads is used. In another embodiment, a 1:5 CD3:CD28 ratio of antibody bound to beads is used. In another embodiment, a 1:4 CD3:CD28 ratio of antibody bound to beads is used. In another embodiment, a 1:3 CD3:CD28 ratio of antibody bound to the beads is used. In yet another embodiment, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particle to cells may depend on particle size relative to the target cell. For example, small sized beads could only bind a few cells, while larger beads could bind many. In certain embodiments the ratio of cells to particles ranges from 1:100 to 100:1 1 and any integer values in-between and in further embodiments the ratio comprises 1:50 to 50:1 and any integer values in between. In another embodiment, the ratio of cells to particles ranges from 1:9 to 9:1 and any integer values in between, can also be used to stimulate T cells. The ratio of anti-CD3-and anti-CD28-coupled particles to T cells that result in T cell stimulation can vary as noted above, however certain preferred values include at least 1:150, 1:125, 1:100, 1:75, 1:50, 1:40, 1:30, 1:20, 1:10, 1:9, 1:8, 1:7, 1:6, 1:5, 1:4, 1:3, 1:2.5, 1:2, 1:1, 2:1, 3:1, 4:1, 5:1, 6:1, 7:1, 8:1, 9:1, 10:1, and 15:1, with one preferred ratio being at least 1:1 particles per T cell. In one particular embodiment, the preferred ratio of particles to cells is 1:5 or 1:10. In one embodiment, a ratio of particles to cells of 1:1 or less is used.

In further embodiments, the ratio of particles to cells can be varied depending on the day of stimulation. For example, in one embodiment, the ratio of particles to cells is from 1:1 to 10:1 1 on the first day and additional particles are added to the cells every day or every other day thereafter for up to 10 days, at final ratios of from 1:1 to 1:10 (based on cell counts on the day of addition). In another embodiment, the ratio of particles to cells is at least about 1:2.5 on the first day and additional particles are added to the cells on day 5 at about 1:10, 1:25, 1:50 or 1:100, on day 7 at 1:10, 1:25, 1:50, or 1:100 and on day 9 at 1:10, 1:25, 1:50, or 1:100. In one particular embodiment, the ratio of particles to cells is 1:1 on the first day of stimulation and adjusted to 1:5 on the third and fifth days of stimulation. In another embodiment, particles are added on a daily or every other day basis to a final ratio of 1:1 on the first day, and 1:5 on the third and fifth days of stimulation. In another embodiment, the ratio of particles to cells is 2:1 on the first day of stimulation and adjusted to 1:10 on the third and fifth days of stimulation. In another embodiment, particles are added on a daily or every other day basis to a final ratio of 1:1 on the first day, and 1:10 on the third and fifth days of stimulation. One of skill in the art will appreciate that a variety of other ratios may be suitable for use in the present application. In particular, ratios will vary depending on particle size and on cell size and type.

One aspect of the present application stems from the surprising finding that using different bead:cell ratios can lead to different outcomes with respect to expansion of antigen-specific T cells. In particular, bead:cell ratios can be varied to selectively expand or delete antigen-specific (memory) T cells. In one embodiment, the particular bead:cell ratio used selectively deletes antigen-specific T cells. Specifically, high bead:cell ratios, such as about 5:1, 10:1, 15:1, 20:1, 25:1, 30:1, 35:1, 40:1, 45:1, 50:1, and higher, induce deletion of antigen-specific T cells. Without being bound by theory, it is thought that the antigen-specific T cells are sensitized to further stimulation. Stimulation with high bead:cell ratios provides a high concentration of stimulating antibody, leading to over-stimulation of antigen-specific T cells, causing them to die, either by apoptosis or other mechanisms. Thus, in this regard, the bead compositions described herein are functioning as a pro-apoptotic composition. Further, in this regard, as the skilled artisan would appreciate, in certain embodiments, the same composition used as a pro-apoptotic composition (*e.g*., a surface having attached thereto an agent that stimulates a cell surface moiety, such as the bead compositions described herein) is used to expand the remaining mixed population of cells for use in any variety of immunotherapeutic settings as described herein. Using lower bead:cell ratios provides a stimulation signal to antigen-specific T cells that does not over-stimulate, but rather induces rapid proliferation of these cells. In a further embodiment, the particular bead:cell ratio used selectively expands antigen-specific T cells. The skilled artisan would readily appreciate that any ratio can be used as long as the desired expansion or deletion occurs. Therefore, the compositions and methods described herein can be used to expand specific populations of T cells, or to delete specific populations of T cells, for use in any variety of immunotherapeutic settings described herein.

Using certain methodologies it may be advantageous to maintain long-term stimulation of a population of T cells following the initial activation and stimulation, by separating the T cells from the stimulus after a period of about 7 to about 14 days. The rate of T cell proliferation is monitored periodically (*e.g*., daily) by, for example, examining the size or measuring the volume of the T cells, such as with a Coulter Counter. In this regard, a resting T cell has a mean diameter of about 6.8 microns, and upon initial activation and stimulation, in the presence of the stimulating ligand, the T cell mean diameter will increase to over 12 microns by day 4 and begin to decrease by about day 6. When the mean T cell diameter decreases to approximately 8 microns, the T cells may be reactivated and re-stimulated to induce further proliferation of the T cells. Alternatively, the rate of T cell proliferation and time for T cell re-stimulation can be monitored by assaying for the presence of cell surface molecules, such as, CD154, CD54, CD25, CD137, CD134, which are induced on activated T cells.

For inducing long-term stimulation of a population of CD4⁺ and/or CD8⁺ T cells, it may be necessary to reactivate and re-stimulate the T cells with a stimulatory agent such as an anti-CD3 antibody and an anti-CD28 antibody (*e.g.* B-T3, XR-CD28 (Diaclone, Besançon, France)) several times to produce a population of CD4⁺ or CD8⁺ cells increased in number from about 10 to about 1,000-fold the original T cell population. For example, in one embodiment, T cells are stimulated as described for 2-3 times. In further embodiments, T cells are stimulated as described for 4 or 5 times. Using the present methodology, it is possible to achieve T cell numbers from about 100 to about 100,000-fold that have increased polyclonality as compared to prior to stimulation. Moreover, T cells expanded by the method of the present application secrete substantial levels of cytokines (*e.g*., IL-2, IFN-γ, IL-4, GM-CSF and TNF-α) into the culture supernatants. For example, as compared to stimulation with IL-2, CD4⁺ T cells expanded by use of anti-CD3 and anti-CD28 co-stimulation secrete high levels of GM-CSF and TNF-α into the culture medium. These cytokines can be purified from the culture supernatants or the supernatants can be used directly for maintaining cells in culture. Similarly, the T cells expanded by the method of the present application together with the culture supernatant and cytokines can be administered to support the growth of cells *in vivo.*

In one embodiment, T cell stimulation is performed, for example with anti-CD3 and anti-CD28 antibodies co-immobilized on beads (3x28 beads), for a period of time sufficient for the cells to return to a quiescent state (low or no proliferation) (approximately 8-14 days after initial stimulation). The stimulation signal is then removed from the cells and the cells are washed and infused back into the patient. The cells at the end of the stimulation phase are rendered "super-inducible" by the methods of the present application, as demonstrated by their ability to respond to antigens and the ability of these cells to demonstrate a memory-like phenotype, as is evidence by the examples. Accordingly, upon re-stimulation either exogenously or by an antigen *in vivo* after infusion, the activated T cells demonstrate a robust response characterized by unique phenotypic properties, such as sustained CD154 expression, increased cytokine production, etc.

In further embodiments, the cells, such as T cells are combined with agent-coated or conjugated beads, the beads and the cells are subsequently separated, and then the cells are cultured. In an alternative embodiment, prior to culture, the agent-coated or conjugated beads and cells are not separated but are cultured together. In a further embodiment, the beads and cells are first concentrated by application of a force, resulting in cell surface moiety ligation, thereby inducing cell stimulation and/or polarization of the activation signal.

By way of example, when T cells are the target cell population, the cell surface moieties may be ligated by allowing paramagnetic beads to which anti-CD3 and anti-CD28 antibodies are attached (3x28 beads) to contact the T cells prepared. In one embodiment the cells (for example, 10⁴ to 10⁹ T cells) and beads (for example, DYNABEADS® M-450 CD3/CD28 T paramagnetic beads at a ratio of 1:1) are combined in a buffer, preferably PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (*i.e.* 100%) may comprise the target cell of interest. Accordingly, any cell number is within the context of the present application. In certain embodiments, it may be desirable to significantly decrease the volume in which particles and cells are mixed together (*i.e.*, increase the concentration of cells), to ensure maximum contact of cells and particles. For example, in one embodiment, a concentration of about 2 billion cells/ml is used. In another embodiment, greater than 100 million cells/ml is used. In a further embodiment, a concentration of cells of 10, 15, 20, 25, 30, 35, 40, 45, or 50 million cells/ml is used. In yet another embodiment, a concentration of cells from 75, 80, 85, 90, 95, or 100 million cells/ml is used. In further embodiments, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain. For example, using high concentration of cells allows more efficient selection of CD8+ T cells that normally have weaker CD28 expression.

In a related embodiment, it may be desirable to use lower concentrations of cells. By significantly diluting the mixture of T cells and particles, interactions between particles and cells is minimized. This selects for cells that express high amounts of desired antigens to be bound to the particles. For example, CD4+ T cells express higher levels of CD28 and are more efficiently captured and stimulated than CD8+ T cells in dilute concentrations. In one embodiment, the concentration of cells used is about 5 X 10⁶/ml. In other embodiments, the concentration used can be from about 1 X 10⁵/ml to about 1 X 10⁶/ml, and any integer value in between.

The buffer that the cells are suspended in may be any that is appropriate for the particular cell type. When utilizing certain cell types the buffer may contain other components, *e.g.* 1-5% serum, necessary to maintain cell integrity during the process. In another embodiment, the cells and beads may be combined in cell culture media. The cells and beads may be mixed, for example, by rotation, agitation or any means for mixing, for a period of time ranging from one minute to several hours. The container of beads and cells is then concentrated by a force, such as placing in a magnetic field. Media and unbound cells are removed and the cells attached to the beads or other surface are washed, for example, by pumping via a peristaltic pump, and then resuspended in media appropriate for cell culture.

In one embodiment, the mixture may be cultured for 30 minutes to several hours (about 3 hours) to about 14 days or any hourly or minute integer value in between. In another embodiment, the mixture may be cultured for 21 days. In one embodiment of the invention the beads and the T cells are cultured together for about eight days. In another embodiment, the beads and T cells are cultured together for 2-3 days. As described above, several cycles of stimulation may also be desired such that culture time of T cells can be 60 days or more. Conditions appropriate for T cell culture include an appropriate media (*e.g.*, Minimal Essential Media or RPMI Media 1640 or, X-vivo 15, (BioWhittaker)) that may contain factors necessary for proliferation and viability, including serum (*e.g.,* fetal bovine or human serum) or interleukin-2 (IL-2), insulin, or any other additives for the growth of cells known to the skilled artisan. Media can include RPMI 1640, AIM-V, DMEM, MEM, α-MEM, F-12, X-Vivo 15, and X-Vivo 20, with added amino acids and vitamins, either serum-free or supplemented with an appropriate amount of serum (or plasma) or a defined set of hormones, and/or an amount of cytokine(s) sufficient for the growth and expansion of T cells. Antibiotics, *e.g*., penicillin and streptomycin, are included only in experimental cultures, not in cultures of cells that are to be infused into a subject. The target cells are maintained under conditions necessary to support growth, for example, an appropriate temperature (*e.g*., 37° C) and atmosphere (*e.g.,* air plus 5% C0₂).

In one embodiment, bead:cell ratios can be tailored to obtain a desired T cell phenotype. In one particular embodiment, bead:cell ratios can be vaired to selectively expand or delete antigen-specific (memory) T cells. In one embodiment, the particular bead:cell ratio used selectively deletes antigen-specific T cells. In a further embodiment, the particular bead:cell ratio used selectively expands antigen-specific T cells. The skilled artisan would readily appreciate that any ratio can be used as long as the desired expansion or deletion of antigen-specific T cells occurs. Therefore, the compositions and methods described herein can be used to expand specific populations of T cells, or to delete specific populations of T cells, for use in any variety of immunotherapeutic settings described herein.

In another embodiment, the time of exposure to stimulatory agents such as anti-CD3/anti-CD28 (*i.e*., 3x28)-coated beads may be modified or tailored in such a way to obtain a desired T cell phenotype. Alternatively, a desired population of T cells can be selected using any number of selection techniques, prior to stimulation. One may desire a greater population of helper T cells (T_{H}), typically CD4⁺ as opposed to CD8⁺ cytotoxic or regulatory T cells, because an expansion of T_{H} cells could improve or restore overall immune responsiveness. While many specific immune responses are mediated by CD8⁺ antigen-specific T cells, which can directly lyse or kill target cells, most immune responses require the help of CD4⁺ T cells, which express important immune-regulatory molecules, such as GM-CSF, CD40L, and IL-2, for example. Where CD4-mediated help is preferred, a method, such as that described herein, which preserves or enhances the CD4:CD8 ratio could be of significant benefit. Increased numbers of CD4⁺ T cells can increase the amount of cell-expressed CD40L introduced into patients, potentially improving target cell visibility (improved APC function). Similar effects can be seen by increasing the number of infused cells expressing GM-CSF, or IL-2, all of which are expressed predominantly by CD4⁺ T cells. Alternatively, in situations where CD4-help is needed less and increased numbers of CD8⁺ T cells are desirous, the XCELLERATE^{™} approaches described herein can also be utilized, by for example, pre-selecting for CD8⁺ cells prior to stimulation and/or culture. Such situations may exist where increased levels of IFN-γ or increased cytolysis of a target cell is preferred. One may also modify time and type of exposure to stimulatory agents to expand T cells with a desired TCR repertoire, *e.g.* expressing desired Vβ family genes.

To effectuate isolation of different T cell populations, exposure times to the particles may be varied. For example, in one preferred embodiment, T cells are isolated by incubation with 3x28 beads, such as DYNABEADS^{®} M-450, for a time period sufficient for positive selection of the desired T cells. In one embodiment, the time period is about 30 minutes. In a further embodiment, the time period is at least 1, 2, 3, 4, 5, or 6 hours. In yet another preferred embodiment, the time period is 10 to 24 hours or more. In one preferred embodiment, the incubation time period is 24 hours. For isolation of T cells from cancer patients, use of longer incubation times, such as 24 hours, can increase cell yield.

In certain embodiments, stimulation and/or expansion times may be 10 weeks or less, 8 weeks or less, four weeks or less, 2 weeks or less, 10 days or less, or 8 days or less (four weeks or less includes all time ranges from 4 weeks down to 1 day (24 hours) or any value between these numbers). In some embodiments in may be desirable to clone T cells using, for example, limiting dilution or cell sorting, wherein longer stimulation time may be necessary. In some embodiments, stimulation and expansion may be carried out for 6 days or less, 4 days or less, 2 days or less, and in other embodiments for as little as 24 or less hours, and preferably 4-6 hours or less (these ranges include any integer values in between). When stimulation of T cells is carried out for shorter periods of time, the population of T cells may not increase in number as dramatically, but the population will provide more robust and healthy activated T cells that can continue to proliferate *in vivo* and more closely resemble the natural effector T cell pool. As the availability of T cell help is often the limiting factor in antibody responses to protein antigens, the ability to selectively expand or selectively infuse a CD4⁺ rich population of T cells into a subject is extremely beneficial. Further benefits of such enriched populations are readily apparent in that activated helper T cells that recognize antigens presented by B lymphocytes deliver two types of stimuli, physical contact and cytokine production, that result in the proliferation and differentiation of B cells.

In the various embodiments, one of ordinary skill in the art understands removal of the stimulation signal from the cells is dependent upon the type of surface used. For example, if paramagnetic beads are used, then magnetic separation is the feasible option. Separation techniques are described in detail by paramagnetic bead manufacturers' instructions (for example, DYNAL Inc., Oslo, Norway). Furthermore, filtration may be used if the surface is a bead large enough to be separated from the cells. In addition, a variety of transfusion filters are commercially available, including 20 micron and 80 micron transfusion filters (Baxter). Accordingly, so long as the beads are larger than the mesh size of the filter, such filtration is highly efficient. In a related embodiment, the beads may pass through the filter, but cells may remain, thus allowing separation. In one particular embodiment, the biocompatible surface used degrades (*i.e*. is biodegradable) in culture during the exposure period.

Although the antibodies used in the methods described herein can be readily obtained from public sources, such as the American Type Culture Collection (ATCC), antibodies to T cell accessory molecules and the CD3 complex can be produced by standard techniques. Methodologies for generating antibodies for use in the methods of the invention are well-known in the art and are discussed in further detail herein.

### LIGAND IMMOBILIZATION ON A SURFACE

As indicated above, the methods of the present invention preferably use ligands bound to a surface. The surface may be any surface capable of having a ligand bound thereto or integrated into and that is biocompatible, that is, substantially non-toxic to the target cells to be stimulated. The biocompatible surface may be biodegradable or non-biodegradable. The surface may be natural or synthetic, and a synthetic surface may be a polymer. The surface may comprise collagen, purified proteins, purified peptides, polysaccharides, glycosaminoglycans, extracellular matrix compositions, liposomes, or cell surfaces. A polysaccharide may include for example, cellulose, agarose, dextran, chitosan, hyaluronic acid, or alginate. Other polymers may include polyesters, polyethers, polyanhydrides, polyalkylcyanoacryllates, polyacrylamides, polyorthoesters, polyphosphazenes, polyvinylacetates, block copolymers, polypropylene, polytetrafluorethylene (PTFE), or polyurethanes. The polymer may be lactic acid or a copolymer. A copolymer may comprise lactic acid and glycolic acid (PLGA). Non-biodegradable surfaces may include polymers, such as poly(dimethylsiloxane) and poly(ethylene-vinyl acetate). Biocompatible surfaces include for example, glass (*e.g*., bioglass), collagen, chitin, metal, hydroxyapatite, aluminate, bioceramic materials, hyaluronic acid polymers, alginate, acrylic ester polymers, lactic acid polymer, glycolic acid polymer, lactic acid/glycolic acid polymer, purified proteins, purified peptides, or extracellular matrix compositions. Other polymers comprising a surface may include glass, silica, silicon, hydroxyapatite, hydrogels, collagen, acrolein, polyacrylamide, polypropylene, polystyrene, nylon, or any number of plastics or synthetic organic polymers, or the like. The surface may comprise a biological structure, such as a liposome or cell surface. The surface may be in the form of a lipid, a plate, bag, pellet, fiber, mesh, or particle. A particle may include, a colloidal particle, a microsphere, nanoparticle, a bead, or the like. In the various embodiments, commercially available surfaces, such as beads or other particles, are useful (*e.g*., Miltenyi Particles, Miltenyi Biotec, Germany; Sepharose beads, Pharmacia Fine Chemicals, Sweden; DYNABEADS™, Dynal Inc., New York; PURABEADS™, Prometic Biosciences).

When beads are used, the bead may be of any size that effectuates target cell stimulation. In one embodiment, beads are preferably from about 5 nanometers to about 500 µm in size. Accordingly, the choice of bead size depends on the particular use the bead will serve. For example, if the bead is used for monocyte depletion, a small size is chosen to facilitate monocyte ingestion (*e.g*., 1.0 µm and 4.5 µm in diameter or any size that may be engulfed, such as nanometer sizes); however, when separation of beads by filtration is desired, bead sizes of no less than 50 µm are typically used. Further, when using paramagnetic beads, the beads typically range in size from about 2.8 µm to about 500 µm and more preferably from about 2.8 µm to about 50 µm. Lastly, one may choose to use super-paramagnetic nanoparticles which can be as small as about 10⁻⁵ nm. Accordingly, as is readily apparent from the discussion above, virtually any particle size may be utilized.

An agent may be attached, incorporated into, coupled to, or integrated into a surface by a variety of methods known and available in the art. The agent may be a natural ligand, a protein ligand, or a synthetic ligand. The attachment may be covalent or noncovalent, electrostatic, or hydrophobic and may be accomplished by a variety of attachment means, including for example, chemical, mechanical, enzymatic, electrostatic, or other means whereby a ligand is capable of stimulating the cells. The attachment of the agent may be direct or indirect (*e.g*. tethered). For example, the antibody to a ligand first may be attached to a surface (direct attachment), or avidin or streptavidin, or a second antibody that binds the first, may be attached to the surface for binding to a biotinylated ligand (indirect attachment). With respect to cell surfaces, the attachment may be via genetic expression of the agent using any number of technologies known in the art, such as transfection or transduction, etc of an expression vector comprising the coding region of the agent of interest. The antibody to the ligand may be attached to the surface via an anti-idiotype antibody. Another example includes using protein A or protein G, or other non-specific antibody binding molecules, attached to surfaces to bind an antibody. Alternatively, the ligand may be attached to the surface by chemical means, such as cross-linking to the surface, using commercially available cross-linking reagents (Pierce, Rockford, IL) or other means. In certain embodiments, the ligands are covalently bound to the surface. Further, in one embodiment, commercially available tosyl-activated DYNABEADS™ or DYNABEADS™ with epoxy-surface reactive groups are incubated with the polypeptide ligand of interest according to the manufacturer's instructions. Briefly, such conditions typically involve incubation in a phosphate buffer from pH 4 to pH 9.5 at temperatures ranging from 4 to 37 degrees C.

In one aspect, the agent, such as certain ligands may be of singular origin or multiple origins and may be antibodies or fragments thereof while in another aspect, when utilizing T cells, the co-stimulatory ligand is a B7 molecule (*e.g*., B7-1, B7-2). These ligands are coupled to the surface by any of the different attachment means discussed above. The B7 molecule to be coupled to the surface may be isolated from a cell expressing the co-stimulatory molecule, or obtained using standard recombinant DNA technology and expression systems that allow for production and isolation of the co-stimulatory molecule(s) as described herein. Fragments, mutants, or variants of a B7 molecule that retain the capability to trigger a co-stimulatory signal in T cells when coupled to the surface of a cell can also be used. Furthermore, one of ordinary skill in the art will recognize that any ligand useful in the activation and induction of proliferation of a subset of T cells may also be immobilized on beads or culture vessel surfaces or any surface. In addition, while covalent binding of the ligand to the surface is one preferred methodology, adsorption or capture by a secondary monoclonal antibody may also be used. The amount of a particular ligand attached to a surface may be readily determined by flow cytometric analysis if the surface is that of beads or determined by enzyme-linked immunosorbant assay (ELISA) if the surface is a tissue culture dish, mesh, fibers, bags, for example.

In a particular embodiment, the stimulatory form of a B7 molecule or an anti-CD28 antibody or fragment thereof is attached to the same solid phase surface as the agent that stimulates the TCR/CD3 complex, such as an anti-CD3 antibody. In addition to anti-CD3 antibodies, other antibodies that bind to receptors that mimic antigen signals may be used. For example, the beads or other surfaces may be coated with combinations of anti-CD2 antibodies and a B7 molecule and in particular anti-CD3 antibodies and anti-CD28 antibodies.

When coupled to a surface, the agents may be coupled to the same surface (*i.e*., in "cis" formation) or to separate surfaces (*i.e*., in "trans" formation). Alternatively, one agent may be coupled to a surface and the other agent in solution. In one embodiment, the agent providing the co-stimulatory signal is bound to a cell surface and the agent providing the primary activation signal is in solution or coupled to a surface. In a preferred embodiment, the two agents are immobilized on beads, either on the same bead, *i.e.,* "cis," or to separate beads, *i.e*., "trans." By way of example, the agent providing the primary activation signal is an anti-CD3 antibody and the agent providing the co-stimulatory signal is an anti-CD28 antibody; and both agents are co-immobilized to the same bead in equivalent molecular amounts. In one embodiment, a 1:1 ratio of each antibody bound to the beads for CD4⁺ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular embodiment an increase of from about .5 to about 3 fold is observed as compared to the expansion observed using a ratio of 1:1. In one embodiment, the ratio of CD3:CD28 antibody bound to the beads ranges from 100:1 to 1:100 and all integer values there between. In one aspect of the present invention, more anti-CD28 antibody is bound to the particles than anti-CD3 antibody, *i.e*. the ratio of CD3:CD28 is less than one. In certain embodiments of the invention, the ratio of anti CD28 antibody to anti CD3 antibody bound to the beads is greater than 2:1. In one particular embodiment, a 1:200 CD3:CD28 ratio of antibody bound to beads is used. In one particular embodiment, a 1:150 CD3:CD28 ratio of antibody bound to beads is used. In one particular embodiment, a 1:100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:45 CD3:CD28 ratio of antibody bound to beads is used. In another embodiment, a 1:40 CD3:CD28 ratio of antibody bound to beads is used. In another embodiment, a 1:35 CD3:CD28 ratio of antibody bound to beads is used. In another embodiment, a 1:30 CD3:CD28 ratio of antibody bound to beads is used. In another embodiment, a 1:25 CD3:CD28 ratio of antibody bound to beads is used. In another embodiment, a 1:20 CD3:CD28 ratio of antibody bound to beads is used. In another embodiment, a 1:15 CD3:CD28 ratio of antibody bound to beads is used. In one preferred embodiment, a 1:10 CD3:CD28 ratio of antibody bound to beads is used. In another embodiment, a 1:5 CD3:CD28 ratio of antibody bound to beads is used. In another embodiment, a 1:4 CD3:CD28 ratio of antibody bound to beads is used. In another embodiment, a 1:3 CD3:CD28 ratio of antibody bound to the beads is used. In yet another embodiment, a 3:1 CD3:CD28 ratio of antibody bound to the beads is used.

### SURFACE-ASSOCIATED AGENTS

Agents contemplated include protein ligands, natural ligands, and synthetic ligands. Agents that can bind to cell surface moieties, and under certain conditions, cause ligation and aggregation that leads to signaling include, but are not limited to, lectins (for example, phyotohaemagluttinin (PHA), lentil lectins, concanavalin A), antibodies, antibody fragments, peptides, polypeptides, glycopeptides, receptors, B cell receptor and T cell receptor ligands, MHC-peptide dimers or tetramers, extracellular matrix components, steroids, hormones (for example, growth hormone, corticosteroids, prostaglandins, tetra-iodo thyronine), bacterial moieties (such as lipopolysaccharides), mitogens, superantigens and their derivatives, growth factors, cytokines, adhesion molecules (such as, L-selectin, LFA-3, CD54, LFA-1), chemokines, and small molecules. The agents may be isolated from natural sources such as cells, blood products, and tissues, or isolated from cells propogated *in vitro,* prepared recombinantly, by chemical synthesis, or by other methods known to those with skill in the art.

In one aspect, when it is desirous to stimulate T cells, useful agents include ligand that are capable of binding the CD3/TCR complex, CD2, and/or CD28 and initiating activation or proliferation, respectively. Accordingly, the term ligand includes those proteins that are the "natural" ligand for the cell surface protein, such as a B7 molecule for CD28, as well as artificial ligands such as antibodies directed to the cell surface protein. Such antibodies and fragments thereof may be produced in accordance with conventional techniques, such as hybridoma methods and recombinant DNA and protein expression techniques. Useful antibodies and fragments may be derived from any species, including humans, or may be formed as chimeric proteins, which employ sequences from more than one species.

Methods well known in the art may be used to generate antibodies, polyclonal antisera, or monoclonal antibodies that are specific for a ligand. Antibodies also may be produced as genetically engineered immunoglobulins (Ig) or Ig fragments designed to have desirable properties. For example, by way of illustration and not limitation, antibodies may include a recombinant IgG that is a chimeric fusion protein having at least one variable (V) region domain from a first mammalian species and at least one constant region domain from a second distinct mammalian species. Most commonly, a chimeric antibody has murine variable region sequences and human constant region sequences. Such a murine/human chimeric immunoglobulin may be "humanized" by grafting the complementarity determining regions (CDRs), which confer binding specificity for an antigen, derived from a murine antibody into human-derived V region framework regions and human-derived constant regions. Antibodies containing CDRs of different specificities can also be combined to generate multi-specific (bi or tri-specific, etc.) antibodies. Fragments of these molecules may be generated by proteolytic digestion, or optionally, by proteolytic digestion followed by mild reduction of disulfide bonds and alkylation, or by recombinant genetic engineering techniques.

Antibodies are defined to be "immunospecific" if they specifically bind the antigen with an affinity constant, Kₐ, of greater than or equal to about 10⁴ M⁻¹, preferably of greater than or equal to about 10⁵ M⁻¹, more preferably of greater than or equal to about 10⁶ M⁻¹, and still more preferably of greater than or equal to about 10⁷ M⁻¹. Affinities of binding partners or antibodies can be readily determined using conventional techniques, for example, those described by Scatchard et al. (Ann. N.Y. Acad. Sci. USA 51:660, 1949) or by surface plasmon resonance (BIAcore, Biosensor, Piscataway, NJ) *See, e.g.,* Wolff et al., Cancer Res., 53:2560-2565, 1993).

Antibodies may generally be prepared by any of a variety of techniques known to those having ordinary skill in the art (*See, e.g*., Harlow et al., Antibodies: A Laboratory Manual, 1988, Cold Spring Harbor Laboratory). In one such technique, an animal is immunized with the ligand as antigen to generate polyclonal antisera. Suitable animals include rabbits, sheep, goats, pigs, cattle, and may include smaller mammalian species, such as, mice, rats, and hamsters. Antibodies of the present invention may also be generated as described in U.S. Patent Nos: 6,150,584, 6,130,364, 6,114,598, 5,833,985, 6,071,517, 5,756,096, 5,736,137, and 5,837,243.

An immunogen may be comprised of cells expressing the ligand, purified or partially purified ligand polypeptides or variants or fragments thereof, or ligand peptides. Ligand peptides may be generated by proteolytic cleavage or may be chemically synthesized. Peptides for immunization may be selected by analyzing the primary, secondary, or tertiary structure of the ligand according to methods know to those skilled in the art in order to determine amino acid sequences more likely to generate an antigenic response in a host animal (*See, e.g.,* Novotny, Mol. Immunol. 28:201-207, 1991; Berzoksky, Science 229:932-40, 1985).

Preparation of the immunogen may include covalent coupling of the ligand polypeptide or variant or fragment thereof, or peptide to another immunogenic protein, such as, keyhole limpet hemocyanin or bovine serum albumin. In addition, the peptide, polypeptide, or cells may be emulsified in an adjuvant (*See* Harlow et al., Antibodies: A Laboratory Manual, 1988 Cold Spring Harbor Laboratory). In general, after the first injection, animals receive one or more booster immunizations according to a preferable schedule for the animal species. The immune response may be monitored by periodically bleeding the animal, separating the sera, and analyzing the sera in an immunoassay, such as an Ouchterlony assay, to assess the specific antibody titer. Once an antibody titer is established, the animals may be bled periodically to accumulate the polyclonal antisera. Polyclonal antibodies that bind specifically to the ligand polypeptide or peptide may then be purified from such antisera, for example, by affinity chromatography using protein A or using the ligand polypeptide or peptide coupled to a suitable solid support.

Monoclonal antibodies that specifically bind ligand polypeptides or fragments or variants thereof may be prepared, for example, using the technique of Kohler and Milstein (Nature, 256:495-497, 1975; Eur. J. Immunol. 6:511-519, 1976) and improvements thereto. Hybridomas, which are immortal eucaryotic cell lines, may be generated that produce antibodies having the desired specificity to a ligand polypeptide or variant or fragment thereof. An animal-for example, a rat, hamster, or preferably mouse-is immunized with the ligand immunogen prepared as described above. Lymphoid cells, most commonly, spleen cells, obtained from an immunized animal may be immortalized by fusion with a drug-sensitized myeloma cell fusion partner, preferably one that is syngeneic with the immunized animal. The spleen cells and myeloma cells may be combined for a few minutes with a membrane fusion-promoting agent, such as polyethylene glycol or a nonionic detergent, and then plated at low density on a selective medium that supports the growth of hybridoma cells, but not myeloma cells. A preferred selection media is HAT (hypoxanthine, aminopterin, thymidine). After a sufficient time, usually about 1 to 2 weeks, colonies of cells are observed. Single colonies are isolated, and antibodies produced by the cells may be tested for binding activity to the ligand polypeptide or variant or fragment thereof. Hybridomas producing antibody with high affinity and specificity for the ligand antigen are preferred. Hybridomas that produce monoclonal antibodies that specifically bind to a ligand polypeptide or variant or fragment thereof are contemplated by the present invention.

Monoclonal antibodies may be isolated from the supernatants of hybridoma cultures. An alternative method for production of a murine monoclonal antibody is to inject the hybridoma cells into the peritoneal cavity of a syngeneic mouse. The mouse produces ascites fluid containing the monoclonal antibody. Contaminants may be removed from the antibody by conventional techniques, such as chromatography, gel filtration, precipitation, or extraction.

Human monoclonal antibodies may be generated by any number of techniques. Methods include but are not limited to, Epstein Barr Virus (EBV) transformation of human peripheral blood cells (*see*, U. S. Patent No. 4,464,456), *in* vitro immunization of human B cells (*see, e.g*., Boerner et al., J. Immunol. 147:86-95, 1991), fusion of spleen cells from immunized transgenic mice carrying human immunoglobulin genes and fusion of spleen cells from immunized transgenic mice carrying immunoglobulin genes inserted by yeast artificial chromosome (YAC) *(see, e.g.,* U. S. Patent No. 5,877,397; Bruggemann et al., Curr. Opin. Biotechnol. 8:455-58, 1997; Jakobovits et al., Ann. N. Y. Acad. Sci. 764:525-35, 1995), or isolation from human immunoglobulin V region phage libraries.

Chimeric antibodies and humanized antibodies for use in the present invention may be generated. A chimeric antibody has at least one constant region domain derived from a first mammalian species and at least one variable region domain derived from a second distinct mammalian species (*See, e.g.,* Morrison et al., Proc. Natl. Acad. Sci. USA, 81:6851-55, 1984). Most commonly, a chimeric antibody may be constructed by cloning the polynucleotide sequences that encode at least one variable region domain derived from a non-human monoclonal antibody, such as the variable region derived from a murine, rat, or hamster monoclonal antibody, into a vector containing sequences that encode at least one human constant region. *(See, e.g.,* Shin et al., Methods Enzymol. 178:459-76, 1989; Walls et al., Nucleic Acids Res. 21:2921-29, 1993). The human constant region chosen may depend upon the effector functions desired for the particular antibody. Another method known in the art for generating chimeric antibodies is homologous recombination (U.S. Patent No. 5,482,856). Preferably, the vectors will be transfected into eukaryotic cells for stable expression of the chimeric antibody.

A non-human/human chimeric antibody may be further genetically engineered to create a "humanized" antibody. Such an antibody has a plurality of CDRs derived from an immunoglobulin of a non-human mammalian species, at least one human variable framework region, and at least one human immunoglobulin constant region. Humanization may yield an antibody that has decreased binding affinity when compared with the non-human monoclonal antibody or the chimeric antibody. Those having skill in the art, therefore, use one or more strategies to design humanized antibodies.

Within certain embodiments, the use of antigen-binding fragments of antibodies may be preferred. Such fragments include Fab fragments or F(ab')₂ fragments, which may be prepared by proteolytic digestion with papain or pepsin, respectively. The antigen binding fragments may be separated from the Fc fragments by affinity chromatography, for example, using immobilized protein A or immobilized ligand polypeptide or a variant or a fragment thereof. An alternative method to generate Fab fragments includes mild reduction of F(ab')2 fragments followed by alkylation *(See, e.g.,* Weir, Handbook of Experimental Immunology, 1986, Blackwell Scientific, Boston).

Non-human, human, or humanized heavy chain and light chain variable regions of any of the above described Ig molecules may be constructed as single chain Fv (sFv) fragments (single chain antibodies). *See, e.g.,* Bird et al., Science 242:423-426, 1988; Huston et al., Proc. Natl. Acad Sci. USA 85:5879-5883, 1988. Multifunctional fusion proteins may be generated by linking polynucleotide sequences encoding an sFv in-frame with polynucleotide sequences encoding various effector proteins. These methods are known in the art, and are disclosed, for example, in EP-B1-0318554, U.S. Patent No. 5,132,405, U.S. Patent No. 5,091,513, and U.S. Patent No. 5,476,786.

An additional method for selecting antibodies that specifically bind to a ligand polypeptide or variant or fragment thereof is by phage display (*See, e.g.,* Winter et al., Annul. Rev. Immunol. 12:433-55,1994; Burton et al., Adv. Immunol. 57:191-280, 1994). Human or murine immunoglobulin variable region gene combinatorial libraries may be created in phage vectors that can be screened to select Ig fragments (Fab, Fv, sFv, or multimers thereof) that bind specifically to a ligand polypeptide or variant or fragment thereof (*See, e.g.,* U.S. Patent No. 5,223,409; Huse et al., Science 246:1275-81, 1989; Kang et al., Proc. Natl. Acad. Sci. USA 88:4363-66, 1991; Hoogenboom et al., J. Molec. Biol. 227:381-388, 1992; Schlebusch et al., Hybridoma 16:47-52, 1997 and references cited therein).

### METHODS OF USE

Generally, the compositions and methodologies described herein can be used to eliminate at least a portion of undesired clonal populations of T cells from a population of immune cells. The methods of the present invention are also used to selectively expand a population of cells that have been deleted for undesired clonal populations for use in the treatment of immune defects associated with hematopoietic stem cell transplantation (including allotransplantation and autotransplantation from sources that include blood, cord blood, and bone marrow), organ transplantation (*e.g*., acute or chronic GVHD), and autoimmune diseases, including autoimmune disease caused by cancers such as large granular lymphocyte (LGL) leukemia, chronic lymphocytic leukemia (CLL) or by common variable immunodeficiency. As a result, a population of cells, in the case of T cells, that express TCRs that are polyclonal with respect to antigen reactivity, but essentially homogeneous with respect to either CD4⁺ or CD8⁺, can be produced that have been cleared of any undesired subpopulations of cells, such as autoreactive cells or alloreactive cells. In addition, the method allows for the expansion of the resulting population of T -cells in numbers sufficient to reconstitute an individual's total CD4⁺ or CD8⁺ T cell population (the population of lymphocytes in an individual is approximately 5 X 10¹¹ cells). The resulting cell population can also be genetically transduced using a variety of techniques known to the skilled artisan and used for immunotherapy.

The major problem in hematopoietic stem cell transplantation is graft-versus-host disease (GVHD), which is caused by alloreactive T cells present in the infused hematopoietic stem cell preparation. Thus, the present invention may be used to remove alloreative T cells and to expand the remaining T cell population for infusion into the patient. The cell compositions of the present invention can be used alone or in conjunction with other therapies.

The T cell compositions created by the present invention may be used in the context of any autoimmune disease. Illustrative autoimmune diseases include, but are not limited to, systemic lupus erythematosus

(SLE), multiple sclerosis (MS), rheumatoid arthritis, progressive systemic sclerosis, Sjogren's syndrome, multiple sclerosis, polymyositis, dermatomyositis, uveitis, arthritis, Type I insulin-dependent diabetes, Hashimoto's thyroiditis, Grave's thyroiditis, myasthenia gravis, autoimmune myocarditis, vasculitis, aplastic anemia, autoimmune hemolytic anemia, myelodysplastic syndrome, Evan's syndrome, stiff person syndrome, atopic dermatitis, psoriasis, Behchet's syndrome, Crohn's disease, biliary cirrhosis, inflammatory bowel disease, ulcerative colitis, Goodpasture's syndrome, Wegener's granulomatosis, paroxysmal nocturnal hemaglobinuria, myelodysplastic syndrome, allergic disorders such as hay fever, extrinsic asthma, or insect bite and sting allergies, and food and drug allergies.

Further uses of the T cell compositions generated by the present invention may include the treatment and/or prophylaxis of: inflammatory and hyperproliferative skin diseases and cutaneous manifestations of immunologically mediated illnesses, such as, seborrhoeis dermatitis, angioedemas, erythemas, acne, and Alopecia areata; various eye diseases (autoimmune and otherwise); allergic reactions, such as pollen allergies, reversible obstructive airway disease, which includes condition such as asthma (for example, bronchial asthma, allergic asthma, intrinsic asthma, extrinsic asthma and dust asthma), particularly chronic or inveterate asthma (for example, late asthma and airway hyper-responsiveness), bronchitis, allergic rhinitis, and the like; inflammation of mucous and blood vessels.

As noted above, the T cell compositions generated by the present invention may be used in the treatment of immune defects associated with organ transplantation, *e.g*., host versus graft disease. Treatment of immune defects associated with any organ transplantation is contemplated herein. For example, the methods and cells generated by the present invention can be used in the treatment of immune defects associated with kidney, heart, lung, and liver transplantation.

In certain embodiments, the cells generated by the present application are administered to a patient following treatment with an agent such as chemotherapy, radiation, immunosuppressive agents, such as cyclosporine, azathioprine, methotrexate, mycophenolate, and FK506, antibodies, or other immunoablative agents such as CAMPATH, anti-CD3 antibodies, cyclophosphamide, fludarabine, cyclosporine, FK506, rapamycin, mycophenolic acid, steroids, FR901228, and irradiation. These drugs inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993; Isoniemi (supra)). In a further embodiment, the cell compositions are administered to a patient with autoimmune disease following T cell ablative therapy using either chemotherapy agents such as, fludarabine, external-beam radiation therapy (CRT), cyclophosphamide, or antibodies such as OKT3 or CAMPATH. In another embodiment, the cell compositions generated by the present application are administered to a patient with autoimmune disease following B-cell ablative therapy such as agents that react with CD20, *e.g*. Rituxan. The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

In a further aspect, at least a substantial portion of autoreactive cells from a patient are eliminated *in vitro* using the methods of the present invention then further stimulated and expanded and administered to the patient. In a related embodiment, at least a substantial portion of autoreactive cells from a patient are eliminated *in vitro* using the methods of the present application then administered to the patient and expanded *in vivo.* It is envisioned as one aspect that the compositions generated by the present application can be used in conjunction with other therapies available in the art for treatment of autoimmune disease.

In one embodiment, T cells can be stimulated and expanded as described herein induce or enhance responsiveness in an individual who is immunocompromised as a result of treatment associated with hematopoietic stem cell transplantation. The present application provides methods for reducing the risk of, or the severity of, an adverse GVHD effect in a patient who is undergoing a hematopoietic stem cell transplant, comprising administering to the patient a population of T cells of the present application. In one particular embodiment, at least a substantial portion of alloreactive cells present in the donor hematopoietic stem cells are eliminated by the methods of the present application. In a further embodiment, the T cell compositions generated by the present application are administered to a patient undergoing a hematopoietic stem cell transplantation following treatment with chemotherapy agents. In a further embodiment, at least a substantial portion of alloreactive cells from the donor marrow are eliminated *in vitro* using the methods of the present application then further stimulated and expanded and then administered to the patient In a further embodiment, at least a substantial portion of alloreactive cells from the donor marrow are eliminated *in vitro* using the methods of the present application then administered to the patient and expanded *in vivo.* It is envisioned as one aspect that the compositions generated by the present application can be used in conjunction with other therapies available in the art for use in hematopoietic stem cell transplantation, such as administration of G-CSF, IL-2, IL-11, IL-7, IL-12, and antiviral treatments.

In one embodiment, T cells can be stimulated and expanded as described herein to induce or enhance responsiveness in an individual who is immunocompromised as a result of treatment associated with organ transplantation, including but not limited to, kidney, heart, lung, and liver transplantation. In one particular embodiment, at least a substantial portion of alloreactive cells present in the recipient are eliminated by the methods of the present application. Thus, the present application provides methods for reducing the risk of, or the severity of, organ rejection. In a further embodiment, the T cell compositions generated by the present application are administered to a patient undergoing an organ transplant following treatment with chemotherapy agents. In a further embodiment, at least a substantial portion of alloreactive cells from the transplant recipient are eliminated in *vitro* using the methods of the present application then further stimulated and expanded and then administered to the patient. It is envisioned as one aspect that the compositions generated by the present application can be used in conjunction with other therapies available known in the art for use in organ transplantation.

Another embodiment, provides a method for selectively expanding a population of TH1 cells from a population of CD4⁺ T cells. In this method, CD4⁺ T cells are co-stimulated with an anti-CD28 antibody, such as the monoclonal antibody 9.3, inducing secretion of Till-specific cytokines, including IFN-γ, resulting in enrichment of T_{H1} cells over T_{H2} cells.

The present application further provides a method for selectively expanding a population of TH2 cells from a population of CD4⁺ T cells. In this method, CD4⁺ T cells are co-stimulated with an anti-CD28 antibody, such as the monoclonal antibody B-T3, XR-CD28, inducing secretion of T_{H2}-specific cytokines, resulting in enrichment of T_{H2} cells over T_{H1} cells (see for example, Fowler, et al. Blood 1994 Nov 15;84(10):3540-9; Cohen, et al., Ciba Found Symp 1994;187:179-93).

The present application further provides methods for using the instant cell compositions in conjunction with regulatory T cells. Regulatory T cells can be generated using art-recognized techniques as described for example, in Woo, et aL, J Immunol. 2002 May 1;168(9):4272-6; Shevach, E.M., Annu. Rev. Immunol. 2000, 18:423; Stephens, et al., Eur. J. Immunol. 2001, 31:1247; Salomon, et al, Immunity 2000, 12:431; and Sakaguchi, et al., Immunol. Rev. 2001,182:18.

The present application further provides a method for selectively expanding a population of T cells expressing a specific Vβ, Vα, Vγ or Vδ gene. For example, in this method, T cells expressing a particular Vβ, Vα, Vγ or Vδ gene are positively or negatively selected and then further expanded/stimulated according to the methods of the present application. Alternatively, stimulated and expanded T cells expressing a particular Vβ, Vα, Vγ or Vδ gene of interest can be positively or negatively selected and further stimulated and expanded.

In another example, blood is drawn into a stand-alone disposable device directly from the patient that contains a sensitizing composition and or two or more immobilized antibodies (e.g., anti-CD3 and anti-CD28) or other components to 69 stimulate receptors required for T cell activation prior to the cells being administered to the subject (*e.g*., immobilized on plastic surfaces or upon separable microparticles). In one embodiment, the disposable device may comprise a container (*e.g*., a plastic bag, or flask) with appropriate tubing connections suitable for combining/docking with syringes and sterile docking devices. This device will contain a solid surface for immobilization of T cell activation components (*e.g*., anti-CD3 and anti-CD28 antibodies); these may be the surfaces of the container itself or an insert and will typically be a flat surface, an etched flat surface, an irregular surface, a porous pad, fiber, clinically acceptable/safe ferro-fluid, beads, etc.). Additionally when using the stand-alone device, the subject can remain connected to the device, or the device can be separable from the patient. Further, the device may be utilized at room temperature or incubated at physiologic temperature using a portable incubator.

As devices and methods for collecting and processing blood and blood products are well known, one of skill in the art would readily recognize that given the teachings provided herein, that a variety of devices that fulfill the needs set forth above may be readily designed or existing devices modified. Accordingly, as such devices and methods are not limited by the specific embodiments set forth herein, but would include any device or methodology capable of maintaining sterility and which maintains blood in a fluid form in which complement activation is reduced and wherein components necessary for T cell activation (*e.g*., anti-CD3 and anti-CD28 antibodies or ligands thereto) may be immobilized or separated from the blood or blood product prior to administration to the subject. Further, as those of ordinary skill in the art can readily appreciate a variety of blood products can be utilized in conjunction with the devices and methods described herein. For example, the methods and devices could be used to provide rapid activation of T cells from cryopreserved whole blood, peripheral blood mononuclear cells, other cyropreserved blood-derived cells, or cryopreserved T cell lines upon thaw and prior to subject administration. In another example, the methods and devices can be used to boost the activity of a previously *ex vivo* expanded T cell product or T cell line prior to administration to the subject, thus providing a highly activated T cell product. Lastly, as will be readily appreciated the methods and devices above may be utilized for autologous or allogeneic cell therapy simultaneously with the subject and donor.

The methods of the present application may also be utilized with vaccines to enhance reactivity of the antigen and enhance in *vivo* effect. In one embodiment, the compositions generated by the present application are administered to a patient in conjunction with a composition that enhances T cells *in vivo,* for example, IL,-2, IL-4, IL-7, IL-10, IL-12, and IL-15. Further, given that T cells expanded by the present application have a relatively long half-life in the body, these cells could act as perfect vehicles for gene therapy, by carrying a desired nucleic acid sequence of interest and potentially homing to sites of cancer, disease, or infection. Accordingly, the cells expanded by the present application may be delivered to a patient in combination with a vaccine, one or more cytokines, one or more therapeutic antibodies, etc. Virtually any therapy that would benefit by a more robust T cell population is within the context of the methods of use described herein.

A variety of in vitro and animal models exist for testing and validating the cell compositions generated by the present application and their applicability to a particular immune system related disease or indication. Accordingly, one of ordinary skill in the art could easily choose the appropriate model from those currently existing in the art. Such models include the use of NOD mice, where IDDM results from a spontaneous T cell dependent autoimmune destruction of insulin-producing pancreatic β cells that intensifies with age (Bottazzo et al., J. Engl. J. Med., 113:353, 1985; Miyazaki et al., Clin. Exp. Immunol., 60:622,1985). In NOD mice, a model of human IDDM, therapeutic strategies that target T cells have been successful in preventing IDDM (Makin et al., Exp. Anim., 29:1, 1980). These include neonatal thymectomy, administration of cyclosporine, and infusion of anti-pan T cell, anti-CD4, or anti-CD25 (IL-2R) monoclonal antibodies (mAbs) (Tarui et al., Insulitis and Type I DiabetesLessons from the NOD Mouse, Academic Press, Tokyo, p.143,1986). Other models include, for example, those typically utilized for autoimmune and inflammatory disease, such as multiple sclerosis (EAE model), rheumatoid arthritis, graft-versus-host disease (transplantation models for studying graft rejection using skin graft, heart transplant, islet of Langerhans transplants, large and small intestine transplants, and the like), asthma models, systemic lupus erythematosus (systemic autoimmunity—lpr or NZBx NZWF1 models), and the like. (see, for example, Takakura et al., Exp. Hematol. 27(12):1815-821, 199; Hu et al., Immunology 98(3):379-385,1999; Blyth et al., Am. J. Respir. Cell Mol. Biol. 14(5):425-438, 1996; Theofilopoulos and Dixon, Adv. Immunol. 37:269-389, 1985; Eisenberg et al., J. Immunol. 125:1032-1036, 1980; Bonneville et al., Nature 344:163-165, 1990; Dent et al., Nature 343:714-719, 1990; Todd et al., Nature 351:542-547, 1991; Watanabe et al., Biochem Genet. 29:325-335, 1991; Morris et al., Clin. Immunol. Immunopathol. 57:263-273,1990; Takahashi et al., Cell 76:969-976, 1994; Current Protocols in Immunology, Richard Coico (Ed.), John Wiley & Sons, Inc., Chapter 15,1998).

Collagen-induced arthritis (CIA) is a T cell dependent animal model of rheumatoid arthritis (RA) (D. E. Trentham et al., "Autoimmunity to Type II Collagen: An Experimental Model of Arthritis," J. Exp. Med.146: 857-868 (1977)). Within two weeks after immunization with type II collage (CII) in IFA, susceptible rats develop polyarthritis with histologic changes of pannus formation and bone/cartilage erosion. In addition, humoral and cellular responses to CII occur in CIA as well as RA (E. Brahn, "animal Models of Rheumatoid Arthritis: Clues to Etiology and Treatment" in Clinical Orthopedics and Related Research (B. Hahn, ed., Philadelphia, JB Lippincott Company, 1991). Consequently, CIA is a useful animal model of RA that serves as an *in vivo* system for the investigation of potentially new therapeutic interventions as described in the present application.

### Pharmaceutical Compositions

T cell populations of the present application may be administered either alone, or as a pharmaceutical composition in combination with diluents and/or with other components such as IL-2 or other cytokines or cell populations. Briefly, pharmaceutical compositions may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (*e.g*., aluminum hydroxide); and preservatives. Composition are preferably formulated for intravenous administration. The present application further provides for pharmaceutical compositions comprising sensitizing compositions as described herein.

Pharmaceutical compositions may be administered in a manner appropriate to the disease to be treated (or prevented). The quantity and frequency of administration will be determined by such factors as the condition of the patient, and the type and severity of the patient's disease, although appropriate dosages may be determined by clinical trials. When "an immunologically effective amount" or "therapeutic amount" is indicated, the precise amount of the compositions of the present invention to be administered can be determined by a physician with consideration of individual differences in age, weight, disease severity and condition of the patient and any other factors relevant to treatment of the patient It can generally be stated that a pharmaceutical composition comprising the subject T cells, may be administered at a dosage of 10⁴ to 10⁷ APC/kg body weight, preferably 10⁵ to 10⁶ APC/kg body weight, including all integer values within those ranges. Cell compositions may also be administered multiple times at these dosages. The cells can be administered by using infusion techniques that are commonly known in immunotherapy (see, *e.g*., Rosenberg et al., New Eng. J. "of Med. 319:1676, 1988). The optimal dosage and treatment regime for a particular patient can readily be determined by one skilled in the art of medicine by monitoring the patient for signs of disease and adjusting the treatment accordingly.

In certain adoptive immunotherapy studies, T cells are administered approximately at 1 X 10⁹ to 2 X 10¹¹ cells to the patient. (See, *e.g.*, U.S. Pat. No. 5,057,423). In some aspects, particularly in the use of allogeneic or xenogeneic cells, lower numbers of cells, in the range of 10⁶/kilogram (10⁶10¹¹ per patient) may be administered. In certain embodiments, T or B cells are administered at 1 X10⁵, 1 X 10⁶, 1 X 10⁷, 1 X 10⁸, 5 X 10⁸, 1 X 10⁹, 5 X 10⁹, 1 X 10¹⁰, 5 X 10¹⁰, 1 X 10¹¹, 5 X 10¹¹, or 1 X 10¹² cells to the subject. T or B cell compositions may be administered multiple times at dosages within these ranges. The T or B cells may be autologous or heterologous (allogeneic or xenogeneic) to the patient undergoing therapy. If desired, the treatment may also include administration of mitogens (*e.g*., PHA) or lymphokines, cytokines, and/or chemokines (*e.g*., GM-CSF, IL-4, IL-13, Flt3-L, RANTES, MIP 1 a, etc.) as described herein to enhance restoration of the immune response.

The compositions generated by the present invention may be useful in methods for preventing, inhibiting,or reducing the severity of autoimmune disease in an animal, which comprise administering to an animal an effective amount of the subject activated polyclonal T cells that have been cleared of undesired subpopulations of autoreactive T cells. The T cell compositions can be administered in conjunction with Tcell ablative therapy and/or other therapies for the treatment of autoimmune diseases.

The compositions generated by the present invention may be useful in providing methods for preventing, inhibiting, or reducing the severity of graft-versus-host disease in an animal requiring a hematopoietic stem cell transplant, which comprise administering to an animal an effective amount of the subject donor bone marrow that has been cleared of undesired subpopulations of alloreactive T cells. The compositions can be administered in conjunction with other therapies for the treatment of immune defects associated with hematopoietic stem cell transplantation.

The compositions generated by the present invention may be useful in providing methods for preventing, inhibiting, or reducing the severity of host-versus-graft disease or graft rejection in an animal requiring an organ transplant, which comprise administering to an animal an effective amount of the subject T cell compositions that has been cleared of undesired subpopulations of alloreactive T cells. The compositions can be administered in conjunction with other therapies for the treatment of immune defects associated with organ transplantation.

The administration of the subject pharmaceutical compositions may be carried out in any convenient manner, including by aerosol inhalation, injection, ingestion, transfusion, implantation or transplantation. The compositions may be administered to a patient subcutaneously, intradermally, intramuscularly, by intravenous (i.v.) injection, intratumorally, or intraperitoneally. Preferably, the T cell compositions are administered by i.v. injection. The compositions of activated T cells may be injected directly into a tumor or lymph node.

In yet another embodiment, the pharmaceutical composition can be delivered in a controlled release system. A pump may be used (see Langer, 1990, Science 249:1527-1533; Sefton 1987, CRC Crit. Ref. Biomed. Eng. 14:201; Buchwald et al., 1980; Surgery 88:507; Saudek et al., 1989, N. Engl. J. Med. 321:574). Polymeric materials can be used (see Medical Applications of Controlled Release, 1974, Langer and Wise (eds.), CRC Pres., Boca Raton, Fla.; Controlled Drug Bioavailability, Drug Product Design and Performance, 1984, Smolen and Ball (eds.), Wiley, New York; Ranger and Peppas, 1983; J. Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105). A controlled release system can be placed in proximity of the therapeutic target, thus requiring only a fraction of the systemic dose (see, *e.g*., Medical Applications of Controlled Release, 1984, Langer and Wise (eds.), CRC Pres., Boca Raton, Fla., vol. 2, pp. 115-138).

The T cell and/or sensitizing composition compositions may also be administered using any number of matrices. Matrices have been utilized for a number of years within the context of tissue engineering (see, *e.g*., Principles of Tissue Engineering (Lanza, Langer, and Chick (eds.)), 1997. Matrices may be used within the novel context of acting as an artificial lymphoid organ to support, maintain, or modulate the immune system, typically through modulation of T cells. Accordingly, one can utilize those matrix compositions and formulations which have demonstrated utility in tissue engineering.

Accordingly, the type of matrix that may be used in the compositions, devices and methods is virtually limitless and may include both biological and synthetic matrices. In one particular example, the compositions and devices set forth by U.S. Patent Nos: 5,980,889; 5,913,998; 5,902,745; 5,843,069; 5,787,900; or 5,626,561 are utilized. Matrices comprise features commonly associated with being biocompatible when administered to a mammalian host. Matrices may be formed from both natural and synthetic materials. The matrices may be non-biodegradable in instances where it is desirable to leave permanent structure or removable strictures in the body of an animal, such as an implant; or biodegradable. The matrices may take the form of sponges, implants, tubes, telfa pads, fibers, hollow fibers, lyophilized components, gels, powder, porous compositions, liposomes, cells, or nanoparticles. In addition, matrices can be designed to allow for sustained release of seeded cells or produced cytokine or other active agent. The matrix may be flexible and elastic, and may be described as a semisolid scaffold that is permeable to substances such as inorganic salts, aqueous fluids and dissolved gaseous agents including oxygen.

A matrix is used herein as an example of a biocompatible substance. Wherever the term matrix or matrices appears these terms should be read to include devices and other substances which allow for cellular retention or cellular traversal, are biocompatible, and are capable of allowing traversal of macromolecules either directly through the substance such that the substance itself is a semi-permeable membrane or used in conjunction with a particular semi-permeable substance.

The following examples are offered by way of illustration, and not by way of limitation.

### EXAMPLE 1

### DELETION OF ANTIGEN-SPECIFIC T CELLS FOLLOWING RESTIMULATION WITH CD3/CD28 XCELLERATE^{™} BEADS

This example describes the elimination of antigen-specific T cells from a mixed population of cells by restimulation with anti CD3/CD28 XCELLERATE^{™} beads (3X28 beads). The generation of XCELLERATED T cells^{™} using the processes described herein is essentially as described in U.S. Patent Application No. 10/133,236.

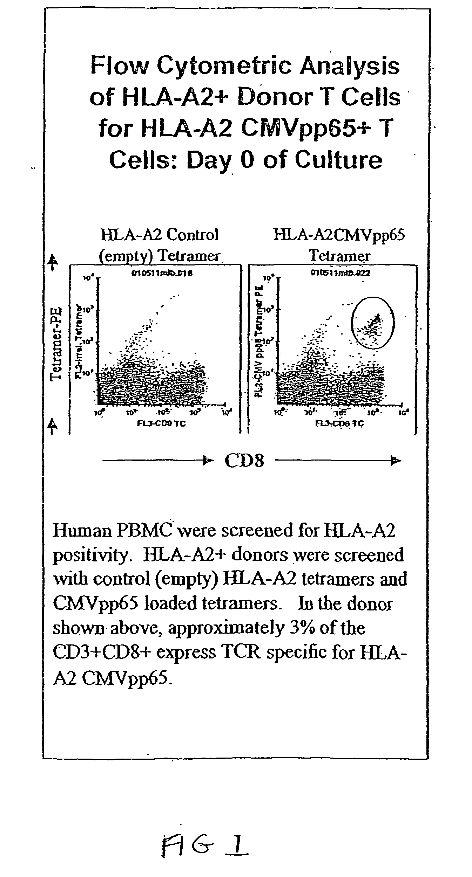

Human PBMC were screened for HLA-A2 CMVpp65 positivity by flow cytometry using HLA-A2 tetramers loaded with CMVpp65 peptide (HLA-A2-CMVpp65). Approximately 3% of the CD3+CD8+ T cells in the donor selected expressed TCR specific for HLA-A2-CMVpp65 (Figure 1).

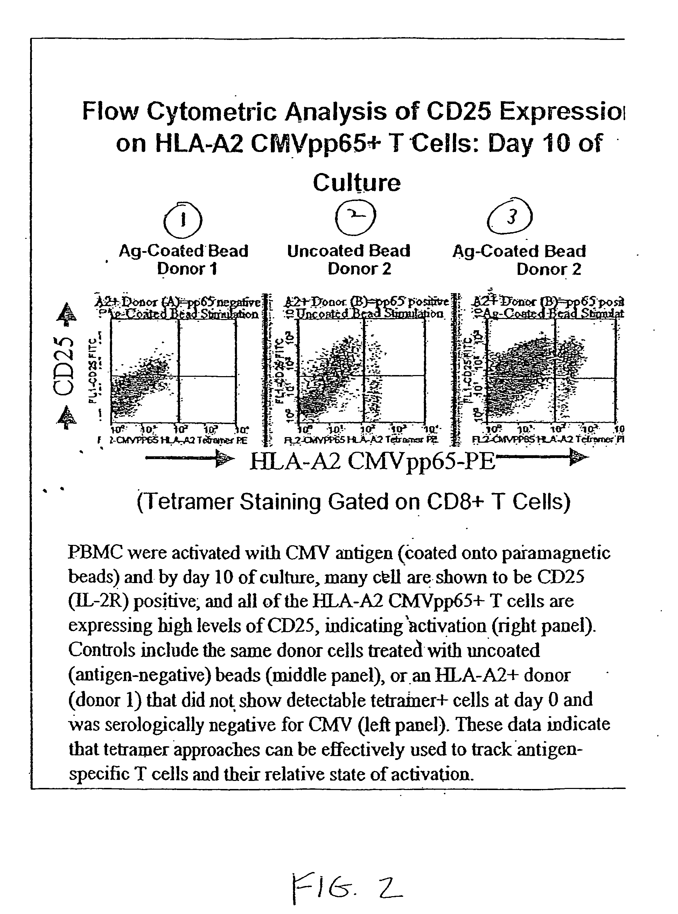

PBMC from the donor (donor 2) and control donor (donor 1) were activated with CMV antigen coated onto paramagnetic beads and by day 10 of culture, many cells were shown by flow cytometric analysis to be CD25 (IL-2R) positive, and all of the HLA-A2 CMVpp65+ T cells expressed high levels of CD25, indicating activation (Figure 2, right panel).

At day 14 post-primary stimulation, cultures were then either left unstimulated (Figure 3, panels A1-A4) or were restimulated using the XCELLERATE^{™} process with 3X28 beads for 16 hours (Figure 3, panels B1-B4). As shown in Figure 3, CD25 is upregulated on restimulated cells (panel B2), but tetramer-positive (*i.e*., CMVpp65-Ag-specific) prestimulated cells were deleted by the secondary strong stimulation provided by the 3X28 beads (panels B3 and B4), while the other cells were unaffected. Similar results were observed when cells were attached to beads or associated with cells attached to beads, magnetically selected and placed back into culture prior to restimulation with the 3X28 beads. In an additional study, the cells were restimulated for an additional 4 days. Deletion of the tetramer-positive cells was still observed after 4 additional days in the 3X28 restimulated cultures.

These results demonstrate that activated CMVpp65-antigen-specific T cells that are restimulated with 3X28 beads are eliminated from the population of cells, most likely through apoptosis.

### EXAMPLE 2

### DETERMINATION OF APOPTOSIS

This example describes an illustrative assay for measuring apoptosis.

DNA Fragmentation Assay: Cells are lysed in 50 µl of lysis buffer (10 mM EDTA, 50 mM Tris pH 8,0.5% sodium dodecyl sulfate, 0.5 mg/ml proteinase K). RNAse A (0.5 mg/ml) is added and lysates are incubated for 1 hr. at 37°C. Two phenol extraction (equal volumes) are performed, followed by one chloroform extraction. DNA is precipitated with two volumes of ice-cold ethanol and incubated at -80°C for 1 hr. DNA is pelleted by centrifugation at 14,000 rpm for 10 minutes at 4°C. Pellets are air-dried for 30 minutes, resuspended in 50 µl of Tris-EDTA pH 8. DNA is electrophoresed in a 1.8% agarose gel in 1 X TBE running buffer (0.05 M Tris base, 0.05 M boric acid, 1 mM disodium EDTA), according to the methods of Preston, et al., Cancer Res., 1994, 54, 4214-4223.

### EXAMPLE 3

### INDUCTION OF APOPTOSIS IN B-CELLS BY COCULTURE WITH XCELLERATED T CELLS™

This example describes the deletion of leukemic B-cells in B-CLL patient samples by co-culture with XCELLERATED T cells^{™}.

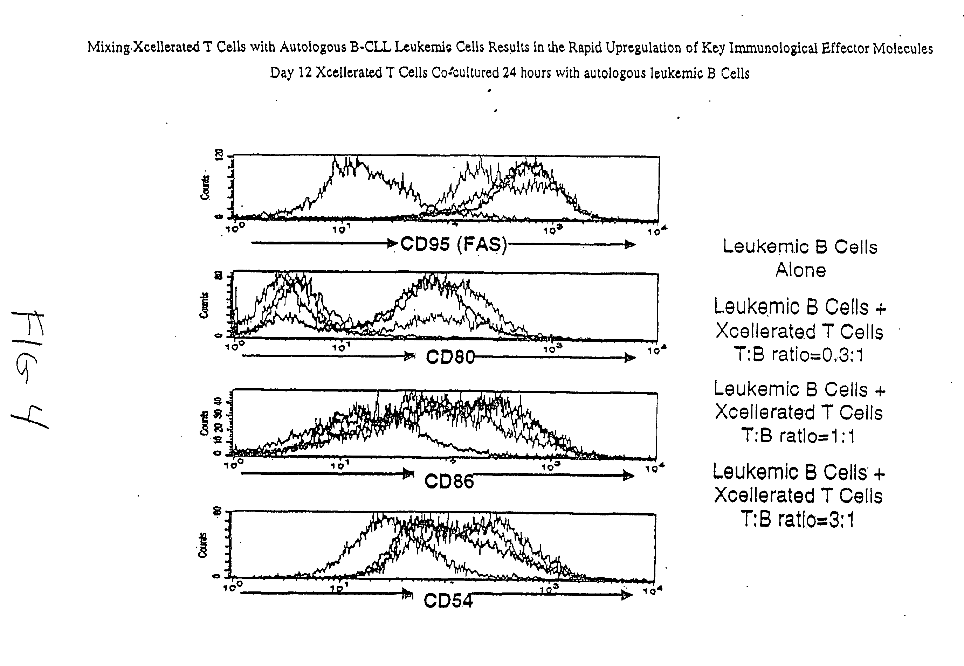

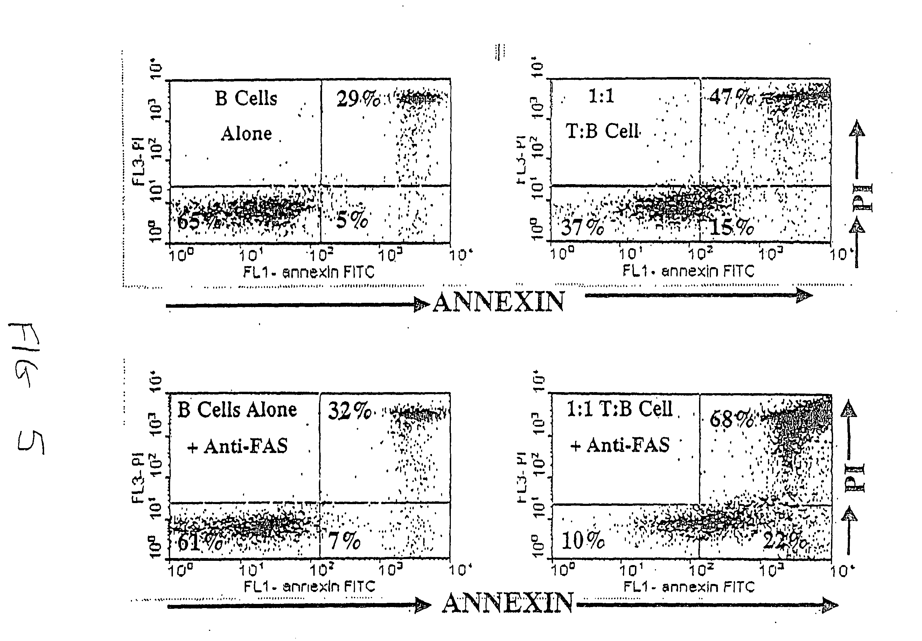

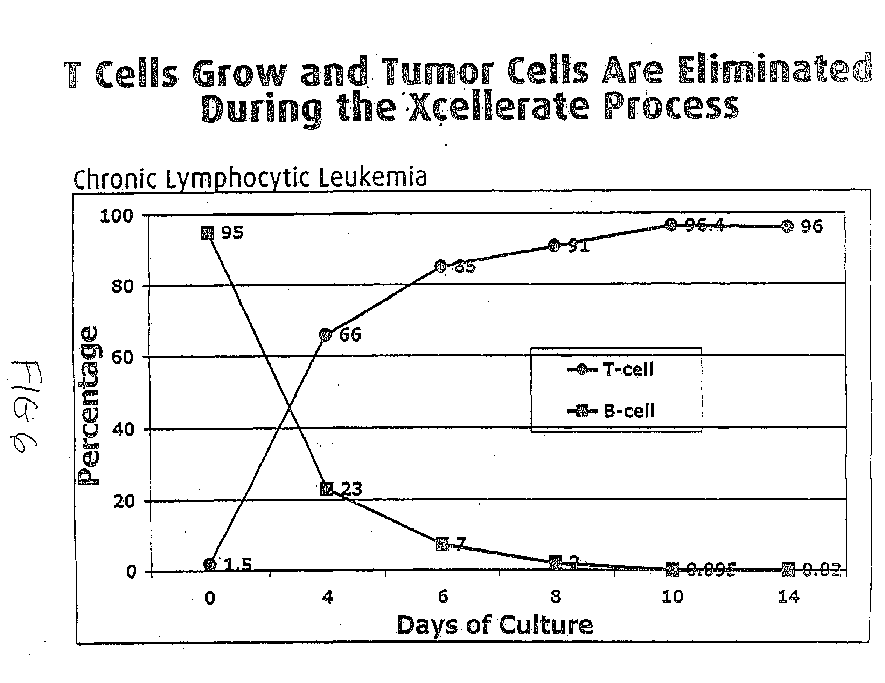

XCELLERATED T cells^{™}, generated essentially as described in U.S. Patent Application No. 10/133,236, were co-cultured with unmanipulated autologous leukemic cells from B-CLL patients. Cell surface markers for CD54, CD80, CD95 (FAS) and CD86, and Annexin/PI (apoptosis) were measured at 24 and 48 hours by flow cytometry. XCELLERATED T cells^{™} were shown to drive up expression of CD95 (FAS) on leukemic B cells (Figure 4). After 48 hours of co-culture with day 12 XCELLERATED T cells^{™}, autologous leukemic B cells show increased expression of CD95 and sensitivity to anti-FAS as measured by flow cytometry (Figure 5). As shown in Figure 5, addition of anti-FAS antibody to co-cultured T:B cells led to increased apoptosis in the leukemic B-cells. In an additional study, it was shown that T cells grow whereas leukemic B-cells are eliminated during the XCELLERATE^{™} process (Figure 6).

In summary, XCELLERATED T cells^{™} upregulate important effector molecules on leukemic B cells, induce functional FAS on leukemic B-cells, and can drive leukemic B-cells into apoptosis pathways. Leukemic B cells were virtually undetectable by the end of the XCELLERATE^{™} process. Therefore, XCELLERATED T cells^{™} can be used as a sensitizing composition or a pro-apoptotic composition for the elimination of leukemic B-cells from a mixed population of cells.

### EXAMPLE 4

### VARYING BEAD:CELL RATIOS CAN SELECTIVELY EXPAND OR DELETE MEMORY CD8 T CELLS

This example shows that the bead:cell ratio can have a profound effect on expansion of different populations of T cells. In particular, a high bead:cell ratio (3:1 - 10:1) tends to induce death in antigen-specific T cells while a lower bead:cell ratio (1:1-1:10) leads to expansion of antigen-specific T cells. Further, the data described below show that lower bead:cell ratios lead to improved cell expansion in polyclonal cell populations as well. Thus, this example shows that lower bead:cell ratios improve overall cell expansion. Further, this example demonstrates that at high bead:cell ratios, the beads described herein can be used as pro-apoptotic compositions.

Cells were prepared and stimulated using the XCELLERATE I™ process essentially as described in U.S. Patent Application No. 10/187,467 filed June 28, 2002. Briefly, in this process, the XCELLERATED T cells^{™} are manufactured from a peripheral blood mononuclear cell (PBMC) apheresis product. After collection from the patient at the clinical site, the PBMC apheresis are washed and cryopreserved. Cells were then thawed, and placed in culture @37°C/5% CO₂ for 1 hour to allow monocytes and other adherent cells to bind to the culture plate. Non-adherent cells were transferred to new culture plates for stimulation as follows. Following this monocyte-depletion step, a volume containing a total of 5 x 10⁸ CD3⁺ T cells is taken and set-up with 1.5 x 10⁹ DYNABEADS® M-450 CD3/CD28 T to initiate the XCELLERATE™ process (approx. 3:1 beads to T cells). The mixture of cells and DYNABEADS^{®} M-450 CD3/CD28 T are then incubated at 37°C, 5% CO₂ for approximately 8 days to generate XCELLERATED T cells^{™} for a first infusion. The remaining monocyte-depleted PBMC are cryopreserved until a second or further cell product expansion (approximately 21 days later) at which time they are thawed, washed and then a volume containing a total of 5 x 10⁸ CD3⁺ T cells is taken and set-up with 1.5 x 10⁹ DYNABEADS^{®} M-450 CD3/CD28 T to initiate the XCELLERATE^{™} Process for a second infusion. During the incubation period of ≈8 days at 37°C, 5% CO₂, the CD3⁺ T cells activate and expand. The anti-CD3 mAb used is BC3 (XR-CD3; Fred Hutchinson Cancer Research Center, Seattle, WA), and the anti-CD28 mAb (B-T3, XR-CD28) is obtained from Diaclone, Besançon, France.

For the experiment described below, cultures containing cells for which adherent cells had been removed then have beads added at bead:T cell ratios as shown in Table 1. The beads used in this Example comprised the DYNABEADS® M-450 CD3/CD28 T with a 1:1 CD3:CD28 antibody ratio bound on the beads.

**Table 1: Varying Bead:Cell Ratios can Selectively Expand or Delete Memory CD8 T cells**

| Bead:Cell Ratio | Fold Increase | |
|---|---|---|
| | Polyclonal T cells | CMV Antigen-Specific T cells |
| 10:1 | 149 | 0 |
| 5:1 | 294 | 0 |
| 3:1 | 346 | 1.4 |
| 1:1 | 562 | 20.6 |
| 1:5 | 113 | 53 |
| 1:10 | 79 | 45.8 |

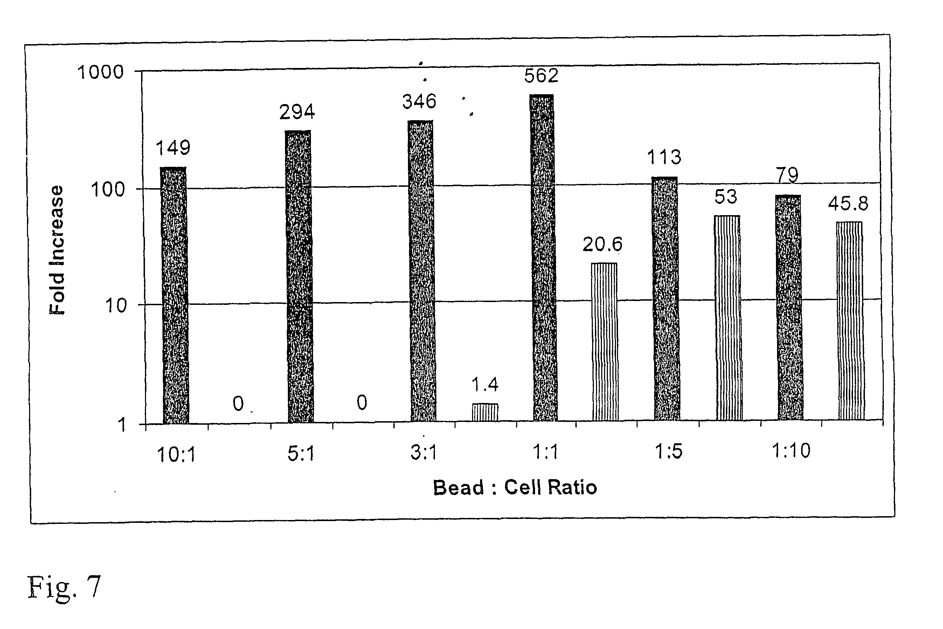

The results summarized in Table 1 and shown graphically in Figure 7 demonstrate that antigen-specific T cells can be selectively deleted by using high bead:cell ratios and expanded using low bead:cell ratios. Similar results were observed with EBV-specific and influenza-specific CD8 T cells (not shown). Without being bound by theory, it is thought that the antigen-specific T cells are sensitized to further stimulation. Stimulation with high bead:cell ratios provides a high concentration of stimulating antibody, leading to over-stimulation of antigen-specific T cells, causing them to die, either by apoptosis or other mechanisms. Thus, in this regard, the beads are functioning as a pro-apoptotic composition. Using lower bead:cell ratios provides a stimulation signal to antigen-specific T cells that does not over-stimulate, but rather induces rapid proliferation of these cells. An increase in proliferation is also observed in the polyclonal population of T cells using lower bead:cell ratios. In particular, the results indicate that a bead:cell ratio of 1:1 is optimal for polyclonal T cell expansion.

Therefore, in this Example, evidence is provided to support the use of differing bead:cell ratios depending on the outcome desired. For expansion of antigen-specific T cells, a lower bead:cell ratio is preferable. If deletion of antigen-specific T cells is the desired outcome, a higher bead:cell ratio is preferable.

### EXAMPLE 5

### DELETION OF ALLO-REACTIVE T CELLS FOLLOWING RESTIMULATION WITH CD3/CD28 XCELLERATE^{™} BEADS

This example describes the deletion of allo-reactive T cells following restimulation with CD3/CD28 XCELLERATE^{™} beads.

PBMC were stimulated for 3 days with either allogeneic PBMC or the JY B-lymphoblastoid allogeneic cell line. On day 3, the allogeneic PBMC- or JY-stimulated PBMC were then cultured with CD3/CD28 beads using the XCELLERATE^{™} process essentially as described in U.S. Patent Application No. 10/350,305, with and without 30 minute positive selection with CD3/CD28 beads. Following the XCELLERATE^{™} process, the cells were then restimulated with either allogeneic PBMC or JY allogeneic antigen and CD25 up-regulation was measured. Restimulation with allogeneic cells following the XCELLERATE^{™} process did not lead to upregulation of CD25 expression (measured using flow cytometric analysis), indicating that the allo-reactive cells had been deleted. In particular, positive selection of JY stimulated CD8+ T cells during the XCELLERATE^{™} process significantly decreased allo-reactivity. However, the T cells remained competent to respond to irrelevant antigens in XCELLERATED^{™} cultures as demonstrated by 3rd party allogeneic PBMC and JY responses (*e.g*., restimulation of JY-stimulated culture with allogeneic PBMC or restimulation of allo-PBMC-stimulated culture with JY).

Thus, these results show that activated allo-reactive T cells are deleted by restimulation with CD3/CD28 beads while the remaining polyclonal T cells can be expanded exponentially for use in any number of immunotherapeutic applications.

## Claims

1. An *ex vivo* method for eliminating at least a substantial portion of a clonal T cell subpopulation from a mixed population of T cells from an individual, comprising exposing a population of cells, wherein at least a portion thereof comprises T cells, to one or more pro-apoptotic or growth inhibiting compositions wherein said exposure induces apoptosis or growth inhibition in at least a substantial portion of at least one clonal T cell population present in the mixed population of T cells; thereby eliminating at least a substantial portion of said clonal T cell population from the mixed population of T cells;
**characterised in that** the pro-apoptotic or growth inhibiting composition comprises an autoantigen.

2. The method of claim 1, further comprising expanding the remaining mixed population of T cells.

3. An *ex vivo* method for eliminating at least a substantial portion of a clonal T cell subpopulation from a mixed population of T cells, comprising:
a. exposing a population of cells wherein at least a portion thereof comprises T cells to one or more compositions that sensitize at least a portion of the T cells to further activation or stimulation,
b. exposing the population of cells to a surface wherein the surface has attached thereto one or more agents that ligate a cell surface moiety of at least a portion of the sensitized T cells and stimulates said sensitized T cells, wherein the exposure of said sensitized T cells to said surface is for a time sufficient to induce apoptosis of said sensitized T cells;
thereby eliminating said sensitized T cells from the population;
**characterised in that** the composition that sensitises comprises an autoantigen.

4. The method of claim 3 wherein step (b) further comprises exposing said population of cells to said surface for a time sufficient to stimulate at least a portion of the remaining cells and wherein said at least a portion of the remaining cells proliferates.

5. The method of claim 2 or claim 4 wherein the remaining mixed population of cells is expanded by exposing said population to a surface, wherein the surface has attached thereto one or more agents that ligate a cell surface moiety of at least a portion of the remaining T cells and stimulates said remaining T cells.

6. The method of claim 5 wherein said surface has attached thereto a first agent that ligates a first T cell surface moiety of a T cell, and the same or a second surface has attached thereto a second agent that ligates a second moiety of said T cell, wherein said ligation by the first and second agent induces proliferation of said T cell.

7. The method of any preceding claim wherein the autoantigen is selected from the group consisting of myelin basic protein (MBP), MBP 84-102, MBP 143-168, pancreatic islet cell antigens, collagen, thyroid antigens, Scl-70, nucleic acid, acetylcholine receptor, S Antigen, and type II collagen.

8. The method of any preceding claim wherein the pro-apoptotic composition comprises allogeneic or xenogeneic cells.

9. The method of any preceding claim wherein the autoantigen comprises one or more compositions selected from the group consisting of: target antigens, nucleic acid molecules, proteins or peptides, and non-protein or non-polynucleotide compounds.

10. The method of claim 2 or claim 4 wherein the exposure of said cells to said surface is for a time sufficient to increase polyclonality.

11. The method of claim 10 wherein the increase in polyclonality comprises a shift from monoclonality to oligoclonality or to polyclonality of the T cell population as measure by a Vβ, Vα, Vγ or Vδ spectratype profile of at least one Vβ, Vα, Vγ or Vδ family gene.

12. The method of any of claims 3 to 11 wherein at least one agent is an antibody or an antigen-binding fragment thereof.

13. The method of claim 6 wherein the first agent is an antibody or an antigen-binding fragment thereof, and the second agent is an antibody or an antigen-binding fragment thereof.

14. The method of claim 13, wherein the first and second agents are different antibodies.

15. The method of claim 13 or claim 14 wherein the first agent is an anti-CD3 antibody, an anti-CD2 antibody, or an antigen-binding fragment of an anti-CD3 or anti-CD2 antibody.

16. The method of any of claims 13 to 15, wherein the second agent is an anti-CD28 antibody or antigen-binding fragment thereof.

17. The method of any of claims 13 to 16, wherein the first agent is an anti-CD3 antibody and the second agent is an anti-CD28 antibody.

18. The method of any preceding claim, further comprising formulating the population of T cells so generated for therapeutic use.

19. The method of claim 3 wherein the composition that sensitises comprises recipient PBMCs that have been treated such that they are unable to continue dividing and the population of T cells comprises donor T cells.

20. The method of claim 3 wherein the composition that sensitises comprises donor cells that have been treated such that they are unable to divide and the population of cells comprises recipient T cells.

## Patentansprüche

1. Ex-vivo-Verfahren für die Eliminierung von mindestens einem substantiellen Teil einer klonalen T-Zell-Subpopulation aus einer gemischten T-Zellpopulation aus einem Individuum, das Folgendes umfasst: Exposition einer mindestens zum Teil aus T-Zellen bestehenden Population von Zellen gegenüber eine m oder mehreren proapoptotischen oder wachstumshemmenden Zusammensetzungen, wobei diese Exposition den programmierten Zelltod oder die Wachstumshemmung in mindestens einem substantiellen Teil von mindestens einer in der gemischten T-Zellpopulation vorhandenen klonalen T-Zellpopulation auslöst und dadurch mindestens einen substantiellen Teil der klonalen T-Zellpopulation aus der gemischten T-Zellpopulation eliminiert;
**dadurch gekennzeichnet, dass** die proapoptotische oder wachstumshemmende Zusammensetzung ein Autoantigen umfasst.

2. Verfahren nach Anspruch 1, das des Weiteren eine Expansion der restlichen gemischten T-Zellpopulation umfasst.

3. Ex-vivo-Verfahren für die Eliminierung von mindestens einem substantiellen Teil einer klonalen T-Zell-Subpopulation aus einer gemischten T-Zellpopulation, das Folgendes umfasst:
a. Exposition einer Population von Zellen, wobei mindestens ein Teil davon T-Zellen umfasst, gegenüber einer oder mehreren Zusammensetzungen, die mindestens einen Teil der T-Zellen sensibilisieren, um eine Aktivierung oder Stimulation zu fördern,
b. Exposition einer Population von Zellen gegenüber einer Oberfläche, wobei an dieser Oberfläche ein Agens oder mehrere Agenzien angeheftet sind, die einen Zelloberflächenrest von mindestens einem Teil der sensibilisierten T-Zellen ligieren und die genannten sensibilisierten T-Zellen stimulieren, wobei die Exposition der sensibilisierten T-Zellen gegenüber der Oberfläche für eine Zeit erfolgt, die ausreicht, um den programmierten Zelltod der sensibilisierten T-Zellen auszulösen;
wodurch die sensibilisierten T-Zellen aus der Population eliminiert werden;
**dadurch gekennzeichnet, dass** die sensibilisierende Zusammensetzung ein Autoantigen umfasst.

4. Verfahren nach Anspruch 3, wobei Schritt (b) des Weiteren Folgendes umfasst: Exposition der Zellpopulation gegenüber der Oberfläche für eine Zeit, die ausreicht, um mindestens einen Teil der restlichen Zellen zu stimulieren und wobei der mindestens eine Teil der restlichen Zellen proliferiert.

5. Verfahren nach Anspruch 2 oder Anspruch 4, wobei die restliche gemischte Zellpopulation expandiert wird, indem die Population gegenüber einer Oberfläche exponiert wird und wobei an dieser Oberfläche ein oder mehrere Agenzien angeheftet sind, die einen Zelloberflächenrest aus mindestens einem Teil der restlichen T-Zellen ligieren und die restlichen T-Zellen stimulieren.

6. Verfahren nach Anspruch 5, wobei sich auf der Oberfläche ein erstes Agens angeheftet ist, das einen ersten T-Zelloberflächenrest einer T-Zelle ligiert, und wobei auf der gleichen oder einer zweiten Oberfläche ein zweites Agens angeheftet ist, das einen zweiten Rest der T-Zelle ligiert, wobei die Ligation des ersten und des zweiten Agens die Proliferation der T-Zelle auslöst.

7. Verfahren nach jedem der vorstehenden Ansprüche, wobei das Autoantigen aus der Gruppe, bestehend aus: Myelin-Basisches Protein (MBP), MBP 84-102, MBP 143-168, pankreatische Inselzellantigene, Collagen, Schilddrüsenantigene, Scl-70, Nukleinsäure, Acetylcholinrezeptor, S-Antigen und Typ-II-Collagen ausgewählt wird.

8. Verfahren nach jedem der vorstehenden Ansprüche, wobei die proapoptotische Zusammensetzung allogene oder xenogene Zellen umfasst.

9. Verfahren nach jedem der vorstehenden Ansprüche, wobei das Autoantigen eine oder mehrere Zusammensetzungen umfasst, die ausgewählt wurden aus der Gruppe bestehend aus: Zielantigene, Nukleinsäuremoleküle, Proteine oder Peptide und proteinfreie oder polynukleotidfreie Verbindungen.

10. Verfahren nach Anspruch 2 oder Anspruch 4, wobei die Exposition der genannten Zellen gegenüber der genannten Oberfläche für eine Zeit erfolgt, die ausreicht, um die Polyklonalität zu steigern.

11. Verfahren nach Anspruch 10, wobei die Steigerung der Polyklonalität einen Wechsel von der Monoklonalität zur Oligoklonalität oder zur Polyklonalität der T-Zellpopulation umfasst, laut Messung durch ein Vβ-, Vα-, Vy- oder Vδ-Spektratypenprofil von mindestens einem Vβ-, Vα-, Vy- oder Vδ-Familiengen.

12. Verfahren nach einem der Ansprüche 3 bis 11, wobei mindestens ein Agens ein Antikörper oder ein antigenbindendes Fragment davon ist.

13. Verfahren nach Anspruch 6, wobei das erste Agens ein Antikörper oder ein antigenbindendes Fragment davon ist, und das zweite Agens ein Antikörper oder antigenbindendes Fragment davon ist.

14. Verfahren nach Anspruch 13, wobei das erste und zweite Agens verschiedene Antikörper sind.

15. Verfahren nach Anspruch 13 oder Anspruch 14, wobei das erste Agens ein Anti-CD3-Antikörper, ein Anti-CD2-Antikörper oder ein antigenbindendes Fragment eines Anti-CD3- oder Anti-CD2-Antikörpers ist.

16. Verfahren nach einem der Ansprüche 13 bis 15, wobei das zweite Agens ein anti-CD28-Antikörper oder ein antigenbindendes Fragment davon ist.

17. Verfahren nach einem der Ansprüche 13 bis 16, wobei das erste Agens ein anti-CD3-Antikörper und das zweite Agens ein anti-CD28-Antikörper ist.

18. Verfahren nach jedem der vorstehenden Ansprüche, des Weiteren umfassend die Formulierung der derart generierten Population von T-Zellen für eine therapeutische Verwendung.

19. Verfahren nach Anspruch 3, wobei die sensibilisierende Zusammensetzung Empfänger-PBMCs umfasst, die so behandelt wurden, dass sie nicht mehr teilungsfähig sind und die T-Zellpopulation Spender-T-Zellen umfasst.

20. Verfahren nach Anspruch 3, wobei die sensibilisierende Zusammensetzung Spenderzellen umfasst, die so behandelt wurden, dass sie nicht mehr teilungsfähig sind und die T-Zellpopulation Empfänger-T-Zellen umfasst.

## Revendications

1. Procédé *ex vivo* destiné à éliminer au moins une partie importante d'une sous-population de lymphocytes T clonaux d'une population mixte de lymphocytes T chez un individu, comprenant l'exposition d'une population de cellules, dont au moins une partie de celle-ci comprend des lymphocytes T, à une ou plusieurs compositions pro-apoptotiques ou inhibitrices de la croissance, ladite exposition induisant une apoptose ou une inhibition de la croissance chez au moins une partie importante d'au moins une population de lymphocytes T clonaux présents dans la population mixte de lymphocytes T ; éliminant ainsi au moins une partie importante de ladite population de lymphocytes T clonaux de la population mixte de lymphocytes T ;
**caractérisé en ce que** la composition pro-apoptotique ou inhibitrice de la croissance comporte un auto-antigène.

2. Procédé de la revendication 1, comprenant en outre l'augmentation de la population mixte restante de lymphocytes T.

3. Procédé *ex vivo* destiné à éliminer au moins une partie importante d'une sous-population de lymphocytes T clonaux d'une population mixte de lymphocytes T, comprenant :
a. l'exposition d'une population de cellules dont au moins une partie comprend des lymphocytes T à une ou plusieurs compositions sensibilisant au moins une partie des lymphocytes T pour mieux les activer ou les stimuler,
b. l'exposition de la population des cellules à une surface, ladite surface ayant, attachés à celle-ci, un ou plusieurs agents qui ligaturent une fraction de surface cellulaire d'au moins une partie des lymphocytes T sensibilisés et stimulant lesdits lymphocytes T sensibilisés, ladite exposition desdits lymphocytes T sensibilisés à ladite surface étant d'une durée de temps suffisante pour induire une apoptose desdits lymphocytes T sensibilisés ;
éliminant ainsi les lymphocytes T sensibilisés de la population ;
**caractérisé en ce que** la composition sensibilisante comprend un auto-antigène.

4. Procédé de la revendication 3 dans lequel l'étape (b) comprend en outre l'exposition de ladite population de cellules à ladite surface pendant une durée de temps suffisante pour stimuler au moins une partie des cellules restantes et dans lequel au moins une partie des cellules restantes prolifère.

5. Procédé de la revendication 2 ou de la revendication 4 dans lequel la population mixte restante de cellules est augmentée en exposant ladite population à une surface, ladite surface ayant, attachés à celle-ci, un ou plusieurs agents qui ligaturent une fraction de surface cellulaire d'au moins une partie des lymphocytes T restants et stimulant lesdits lymphocytes T restants.

6. Procédé de la revendication 5 dans lequel ladite surface, a, attaché à celle-ci, un premier agent qui ligature une première fraction de surface cellulaire d'un lymphocyte T, et la même ou une seconde surface, a, attaché à celle-ci, un second agent qui ligature une seconde fraction dudit lymphocyte T, ladite ligature par le premier et par le second agent induisant une prolifération dudit lymphocyte T.

7. Procédé de l'une quelconque des revendications précédentes dans lequel l'auto-antigène est choisi dans le groupe qui consiste en la protéine basique de la myéline (PBM), la PBM 84-102, la PBM 143-168, les antigènes cellulaires des îlots pancréatiques, le collagène, les antigènes thyroïdiens, le Scl-70, l'acide nucléique, le récepteur de l'acétylcholine, l'antigène S et le collagène de type II.

8. Procédé de l'une quelconque des revendications précédentes dans lequel la composition pro-apoptotique comprend des cellules allogéniques ou xénogéniques.

9. Procédé de l'une quelconque des revendications précédentes dans lequel l'auto-antigène comprend une ou plusieurs compositions choisies dans le groupe qui consiste en des antigènes cibles, des molécules de l'acide nucléique, des protéines ou des peptides et des composés non protéiques ou non polynucléotidiques.

10. Procédé de la revendication 2 ou de la revendication 4 dans lequel l'exposition desdites cellules à ladite surface est d'une durée suffisante pour augmenter la polyclonalité.

11. Procédé de la revendication 10 dans lequel l'augmentation polyclonale comprend un passage de la monoclonalité à l'oligoclonalité ou à la polyclonalité de la population de lymphocytes T mesuré par un profil de type spectral Vβ, Vά, Vγ ou Vδ d'au moins un gène de la famille Vβ, Vά, Vγ ou Vδ.

12. Procédé de l'une des revendications 3 à 11 dans lequel au moins un agent est un anticorps ou un fragment de celui-ci se liant à un antigène.

13. Procédé de la revendication 6 dans lequel le premier agent est un anticorps ou un fragment de celui-ci se liant à un antigène, et le second agent est un anticorps ou un fragment de celui-ci se liant à un antigène.

14. Procédé de la revendication 13 dans lequel le premier et le second agent sont des anticorps différents.

15. Procédé de la revendication 13 ou de la revendication 14 dans lequel le premier agent est un anticorps anti-CD3, un anticorps anti-CD2 ou un fragment se liant à un antigène d'un anticorps anti-CD3 ou d'un anticorps anti-CD2.

16. Procédé de l'une des revendications 13 à 15 dans lequel le second agent est un anticorps anti-CD28 ou un fragment de celui-ci se liant à un antigène.

17. Procédé de l'une des revendications 13 à 16 dans lequel le premier agent est un anticorps anti-CD3 et le second agent est un anticorps anti-CD28.

18. Procédé de l'une quelconque des revendications précédentes, comprenant en outre la formulation de la population de lymphocytes T ainsi générée à des fins thérapeutiques.

19. Procédé de la revendication 3 dans lequel la composition sensibilisante comprend des CMSP réceptrices ayant été traitées de telle sorte qu'elles sont incapables de continuer de se diviser et la population de lymphocytes T comprend des lymphocytes T donneurs.

20. Procédé de la revendication 3 dans lequel la composition sensibilisante comprend des cellules donneuses ayant été traitées de telle sorte qu'elles sont incapables de continuer de se diviser et la population de lymphocytes T comprend des lymphocytes T récepteurs.
